# FASCICULE DE BREVET EUROPEEN

(11) **EP 0 911 317 B1**
(45) Date de publication et mention de la délivrance du brevet: **13.09.2000**
(21) Numéro de dépôt: 98402627.8
(22) Date de dépôt: 22.10.1998
(51) Int. Cl.: C07C 215/68, C07C 213/02, A61K 7/13, C09B 53/00

(54) **Procédé de synthèse de 2-hydroyalkyl paraphénylènediamines, nouvelles 2-hydroxyalkyl paraphénylènediamines, leur utilisation pour la teinture d'oxydation, compositions tinctoriales et procédés de teinture**
Verfahren zur Herstellung von 2-Hydroyalkyl Paraphenylendiaminen, neue 2-Hydroxyalkyl Paraphenylendiamine, deren Verwendung zum oxidativen Färben, Färbemittel und Färbeverfahren
Process for the preparation of 2-hydroyalkyl paraphenylenediamines, novel 2-hydroxyalkyl paraphenylenediamines, their use for oxydative dyeing, dye compositions and dyeing processes

(30) Priorité: 22.10.1997 FR 9713241
(43) Date de publication de la demande: 28.04.1999
(73) Titulaire: L'OREAL, 75008 Paris (FR)
(72) Inventeur: Genet, Alain, 93600 Aulnay-Sous-Bois (FR); Lagrange, Alain, 77700 Coupvray (FR)
(74) Mandataire: Goulard, Sophie

(56) Documents cités:
- EP-A- 0 007 537
- EP-A- 0 722 710
- US-A- 2 273 564

## Description

La présente invention concerne un nouveau procédé de synthèse de 2-hydroxyalkyl paraphénylènediamines substituées ou non substituées en position 5 du noyau benzénique, de nouvelles 2-hydroxyalkyl paraphénylènediamines substituées en position 5 du noyau benzénique, leur utilisation pour la teinture d'oxydation des fibres kératiniques, les compositions tinctoriales les contenant, ainsi que les procédés de teinture les mettant en oeuvre.

Il est connu de teindre les fibres kératiniques et en particulier les cheveux humains avec des compositions tinctoriales contenant des précurseurs de colorant d'oxydation appropriés, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des agents oxydants convenables, peuvent donner naissance par un processus de condensation oxydative à des composés colorés et colorants.

La demande de brevet EP 0 722 710 divulge notamment l'utilisation d'une paraphénylénediamine dans une composition tinctoriale pour fibres kératiniques.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Parmi l'ensemble des bases d'oxydation citées ci-dessus, une famille très largement utilisée est celle des paraphénylènediamines. Les paraphénylènediamines peuvent se présenter sous la forme de composés diversement substitués sur le noyau benzénique, notamment sous la forme de 2-hydroxyalkyl paraphénylènediamines présentant des qualités tinctoriales intéressantes.

Les compositions tinctoriales renfermant ces paraphénylènediamines particulières et éventuellement un ou plusieurs coupleurs permettent en effet d'obtenir des colorations aux nuances variées, dans l'intensité souhaitée, et présentant une bonne tenue face aux agents extérieurs (lumière, intempéries, lavage, ondulation permanente, transpiration, frottements).

Il a déjà été proposé un procédé de synthèse des 2-hydroxyalkyl paraphénylènediamines. Ainsi selon l'enseignement des demandes de brevet FR-A-2 647 341 et DE-A-3 441 148, la synthèse des 2-hydroxyalkyl paraphénylènediamines peut être réalisée par acétylation, nitration en positions 4 et 5 du noyau benzénique, désacétylation, séparation de l'isomère nitré en position 5, et réduction successives de 2-hydroxyalkyl anilines suivant le schéma de synthèse général suivant :

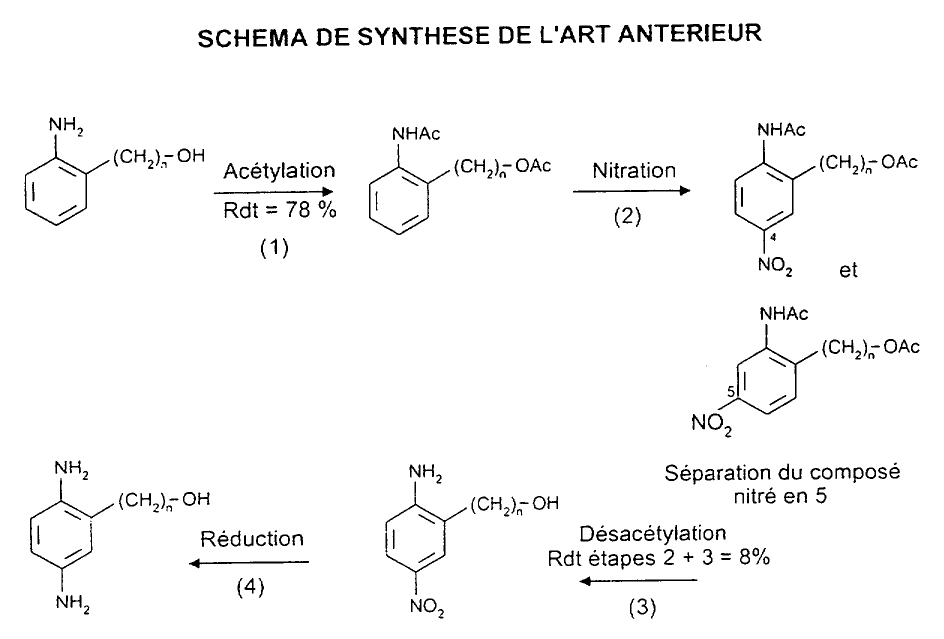

Les rendements figurant sur ce schéma réactionnel concernent les composés dans lesquels n = 2.

Le procédé de synthèse décrit dans ce schéma de l'art antérieur présente cependant un inconvénient majeur, puisque l'étape (2) de nitration conduit à un mélange de composés nitrés en position 4 et de composés nitrés en position 5, et qu'une faible partie des 2-hydroxyalkyl anilines acétylées de départ est transformée en composés nitrés en position 4 du noyau benzénique (soit 8%, après séparation des composés nitrés en position 5, pour les composés dans lesquels n = 2). Ce faible rendement et l'obligation de procéder à une séparation des isomères nitrés ne sont pas acceptables sur un plan industriel.

De plus ce procédé de synthèse de l'art antérieur ne permet pas d'aboutir à des 2-hydroxyalkyl paraphénylènediamines pouvant comporter une substitution en position 5 du noyau benzénique.

C'est en cherchant à remédier à ces problèmes que la demanderesse a découvert le procédé objet de l'invention.

L'invention a donc pour premier objet un nouveau procédé de synthèse de 2-hydroxyalkyl paraphénylènediamines substituées ou non substituées en position 5 du noyau benzénique et de leurs sels d'addition avec un acide, caractérisé par le fait qu'il comprend au moins les étapes suivantes :
Etape n°1 : on protège la fonction amine en position 1 du noyau benzénique et la fonction alcool en position 2 du noyau benzénique d'une 2-hydroxyalkyle aniline substituée en position 5 du noyau benzénique par un atome d'halogène ou par un radical alkyle en C₁-C₆ ;
Etape n°2 : on effectue, sur le composé obtenu à l'étape n° 1, une réaction de nitration en position 4 du noyau benzénique ;
Etape n°3 : on effectue la déprotection de la fonction amine en position 1 du noyau benzénique et de la fonction alcool en position 2 du noyau benzénique du composé obtenu à l'étape n° 2 :
Etape n°4 : on effectue sur le composé obtenu à l'étape n° 3, une réaction de réduction du groupe nitro en position 4, accompagnée ou non, dans le cas d'un composé 5-halogéné, d'une déshalogénation.

Lors de la première étape, les fonctions amine et alcool de la 2-hydroxyalkyl aniline respectivement en position 1 et 2 du noyau benzénique peuvent être protégées par tout radical capable de protéger une fonction amine ou une fonction alcool tel que par exemple un radical alkylcarbonyle, alkylsulfonyle ou phénylsulfonyle.

Le procédé de synthèse de l'invention peut se résumer suivant le schéma de synthèse général suivant : 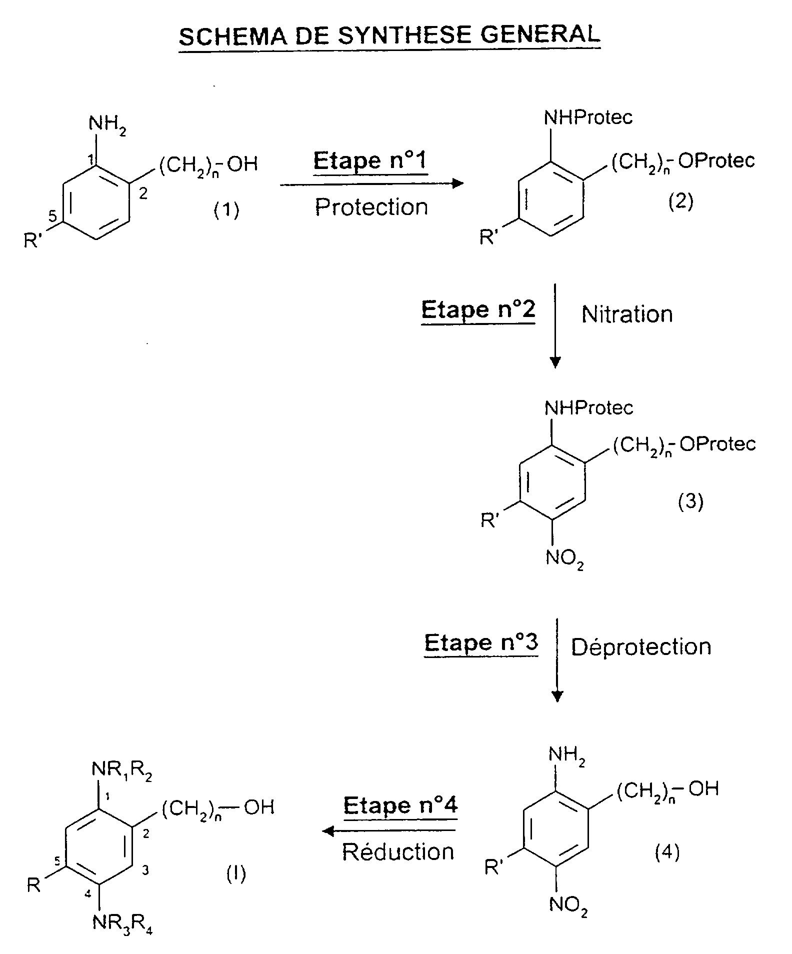 dans lequel
- n est un nombre entier compris entre 1 et 4 inclusivement ;
- Protec signifie un groupement protecteur de la fonction amine et de la fonction alcool ;
- R' représente un atome d'halogène tel que le brome, le chlore, ou le fluor, ou un radical alkyle en C₁-C₆ ;
- R représente un atome d'hydrogène ou a les mêmes significations que R' ;
- R₁, R₂, R₃, R₄ , identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle; carboxyalkyle en C₁-C₆; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
étant entendu qu'au moins un des groupes -NR₁R₂ et -NR₃R₄ désigne un groupement -NH₂.

Selon l'invention, les radicaux alkyle peuvent être linéaires ou ramifiés.

Le procédé de synthèse conforme à l'invention se distingue essentiellement de celui de l'art antérieur par le fait que le composé de départ est une 2-hydroxyalkyl aniline comportant un substituant en position 5 du noyau benzénique, ce qui a pour effet d'orienter la réaction de nitration en position 4 du noyau benzénique et pour conséquence d'augmenter de façon considérable le rendement de la réaction intermédiaire de nitration (Etape n°2).

A titre de comparatif, la Demanderesse a, parallèlement au procédé de synthèse de l'art antérieur décrit ci-dessus pour les composés dans lesquels n = 2, réalisé la synthèse de la 2-β-hydroxyéthyl paraphénylènediamine avec un bon rendement global, à partir de la 5-chloro 2-β-hydroxyéthyl aniline. Le rendement obtenu après réalisation des étapes 2 et 3 telles que décrites ci-dessus pour le procédé de synthèse général conforme à l'invention était de 86,4 %, au lieu de 8 % pour le procédé de synthèse de l'art antérieur.

Les 2-hydroxyalkyl paraphénylènediamines substituées ou non substituées en position 5 sur le noyau benzénique, ainsi que leurs sels d'addition avec un acide pouvant être obtenues selon le procédé conforme à l'invention, répondent de préférence à la formule (I) suivante : 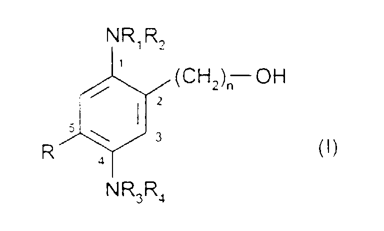 dans laquelle :
- n est un nombre entier compris entre 1 et 4 inclusivement ;
- R désigne un atome d'hydrogène ou d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ;
- R₁, R₂, R₃, R₄, identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle; carboxyalkyle en C₁-C₆ ; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
étant entendu qu'au moins un des groupes -NR₁R₂ et -NR₃R₄ désigne un groupement -NH₂.

Selon une première forme de réalisation du procédé de synthèse conforme à l'invention, les composés particuliers de formule (la) suivante et leurs sels d'addition avec un acide : 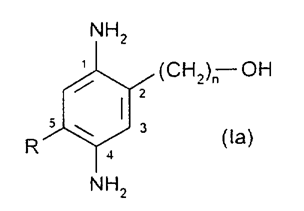 dans lesquels les radicaux R₁, R₂, R₃ et R₄ désignent simultanément un atome d'hydrogène et R et n ont les mêmes significations que celles indiquées ci-dessus pour la formule (I) peuvent être préparés selon le procédé de synthèse A consistant, dans une première étape, à effectuer sur un composé de formule (1) suivante : 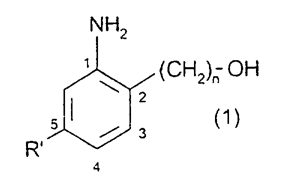 dans laquelle R' désigne un atome d'halogène ou un radical alkyle en C₁-C₆ et n a la même signification que celle indiquée ci-dessus pour la formule (I), une réaction d'acétylation pour obtenir un composé de formule (2) suivante : 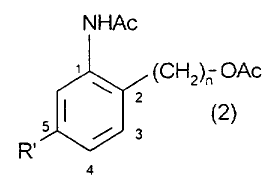 dans laquelle R' et n ont les mêmes significations que celles indiquées ci-dessus pour la formule (1) et Ac représente un groupement acétyle, puis dans une deuxième étape, à effectuer sur le composé de formule (2) ainsi obtenu, une réaction de nitration en position 4 du noyau benzénique, pour obtenir un composé de formule (3) suivante : 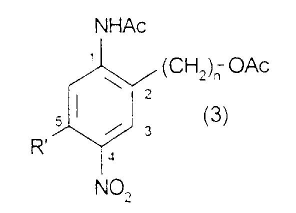 dans laquelle R', n et Ac ont les mêmes significations que celles indiquées ci-dessus pour la formule (2), puis dans une troisième étape, à effectuer sur le composé de formule (3), une réaction de désacétylation pour obtenir le composé de formule (4) suivante : 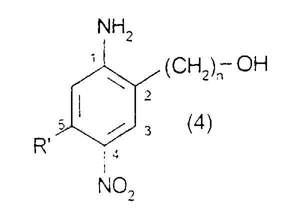 dans laquelle R' et n ont les mêmes significations que celles indiquées ci-dessus pour la formule (3), puis pour obtenir les composés de formule (Ia) dans lesquels R désigne un groupe OZ ou SZ, on fait réagir, dans une étape 3', un composé de formule (4), dans lequel R' désigne un atome d'halogène, avec un alcool de formule HOZ ou un thiol de formule HSZ dans lesquelles Z a les mêmes significations que celles indiquées pour la formule (la), pour obtenir un composé de formule (4') suivante : 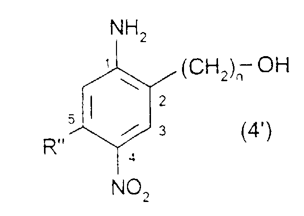 dans laquelle R" désigne un groupe OZ ou SZ, Z et n ayant les mêmes significations que celles indiquées pour la formule (la) ci-dessus, puis dans une quatrième étape, on effectue une réaction de réduction sur le composé de formule (4) ou (4'), étant entendu que lorsqu'on fait réagir le composé de formule (4) dans lequel R' désigne un atome d'halogène, le mode de réduction est choisi parmi les procédés suivants :
- pour obtenir les composés de formule (la) dans laquelle R désigne un atome d'hydrogène, on effectue une réaction de réduction déshalogénante ;
- pour obtenir les composés de formule (la) dans laquelle R désigne un atome d'halogène, on effectue une réaction de réduction non déshalogénante.

Selon une variante de ce procédé de synthèse A conforme à l'invention, on peut aussi faire réagir, dans une étape 2', un composé de formule (3), dans lequel R' désigne un atome d'halogène, avec un alcool de formule HOZ ou un thiol de formule HSZ, Z ayant les mêmes significations que celles indiquées pour la formule (1), pour obtenir un composés de formule (3') suivante : 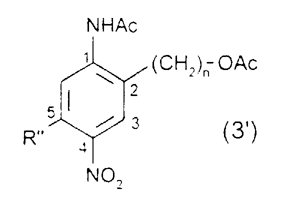 dans laquelle Ac et R" ont les mêmes significations que celles indiquées ci-dessus pour les formules (3) et (4'), puis effectuer une réaction de désacétylation pour obtenir le composé de formule (4') telle que définie ci-dessus.

Ce procédé de synthèse A des composés particuliers de formule (la) peut-être représenté par le schéma A suivant :

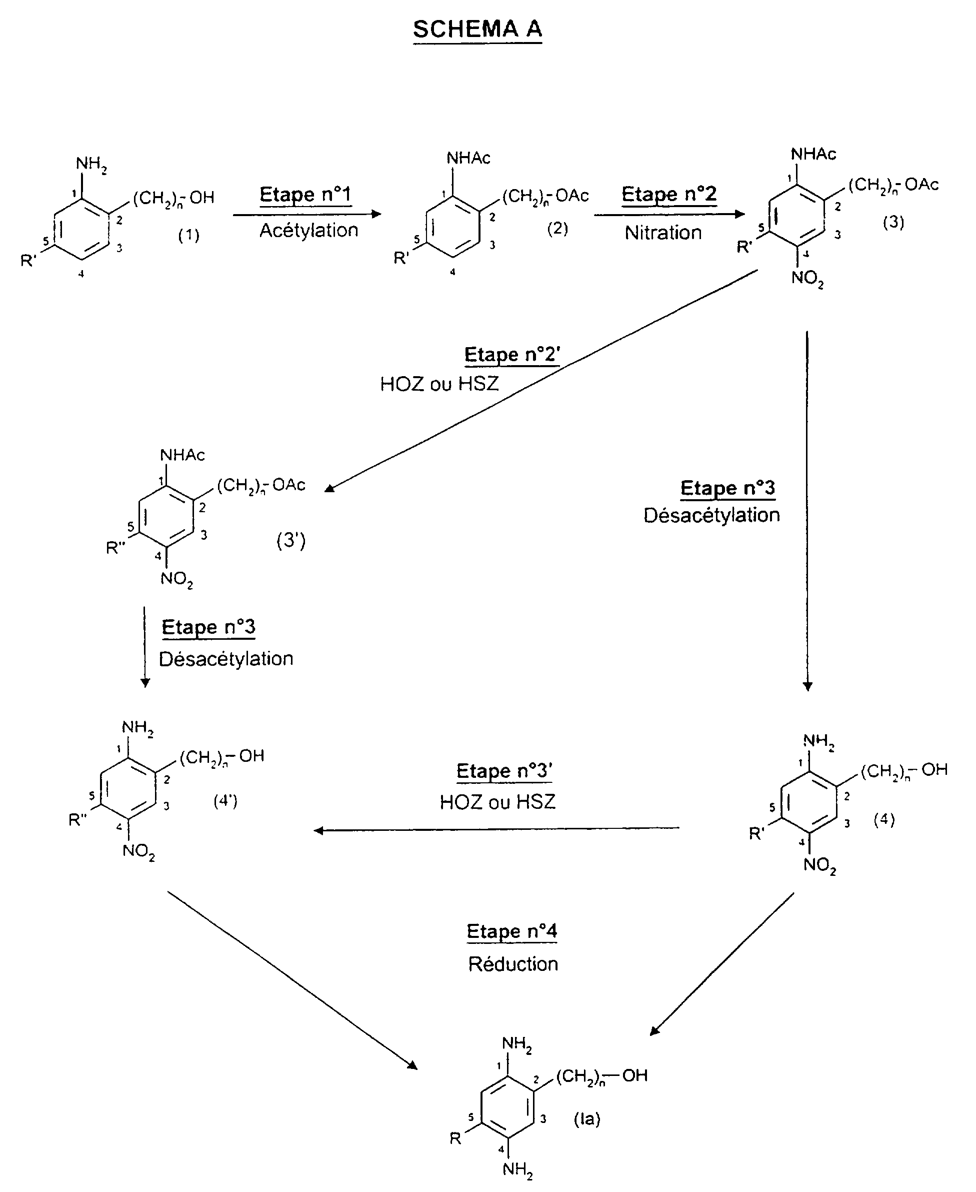

Selon une deuxième forme de réalisation du procédé de synthèse conforme à l'invention, les composés particuliers de formule (Ib) suivante et leurs sels d'addition avec un acide : 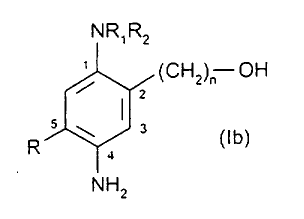 dans laquelle :
- R₁, R₂, R et n ont les mêmes significations que celles indiquées ci-dessus pour la formule (I), étant entendu qu'au moins un des radicaux R₁ et R₂ est différent d'un atome d'hydrogène, peuvent être préparés selon le procédé de synthèse B consistant à réaliser les étapes 1, 2 et 3 du procédé de synthèse A décrit précédemment pour obtenir un composé de formule (4) telle que décrite ci-dessus, puis dans une étape 3", à effectuer sur ledit composé de formule (4) la substitution de la fonction amine en position 1 du noyau benzénique par les radicaux R₁ et/ou R₂, pour obtenir un composé de formule (5) suivante : 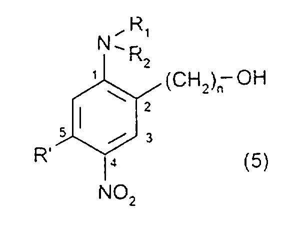 dans laquelle R' et n ont les mêmes significations que celles indiquées ci-dessus pour la formule (3) et R₁ et R₂ ont les mêmes signification que celles indiquées ci-dessus pour la formule (Ib), puis pour obtenir les composés de formule (Ib) dans lesquels R désigne un groupe OZ ou SZ, on fait réagir dans une étape 3' un composé de formule (5), dans lequel R' désigne un atome d'halogène, avec un alcool de formule HOZ ou un thiol de formule HSZ dans lesquelles Z a la même signification que celle indiquée pour la formule (Ib), pour obtenir un composé de formule (5') suivante : 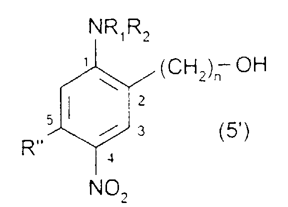 dans laquelle R" désigne un groupe OZ ou SZ, Z et n ayant les mêmes significations que celles indiquées dans la formule (Ib) ci-dessus, puis dans une quatrième étape, on effectue une réaction de réduction sur le composé de formule (5) ou (5'), étant entendu que lorsqu'on fait réagir un composé de formule (5) dans laquelle R' désigne un atome d'halogène, le mode de réduction est choisi parmi les procédés suivants :
- pour obtenir les composés de formule (Ib) dans laquelle R désigne un atome d'hydrogène, on effectue une réaction de réduction déshalogénante ;
- pour obtenir les composés de formule (Ib) dans laquelle R désigne un atome d'halogène, on effectue une réaction de réduction non déshalogénante.

Ce procédé de synthèse B peut être représenté par le schéma B suivant :

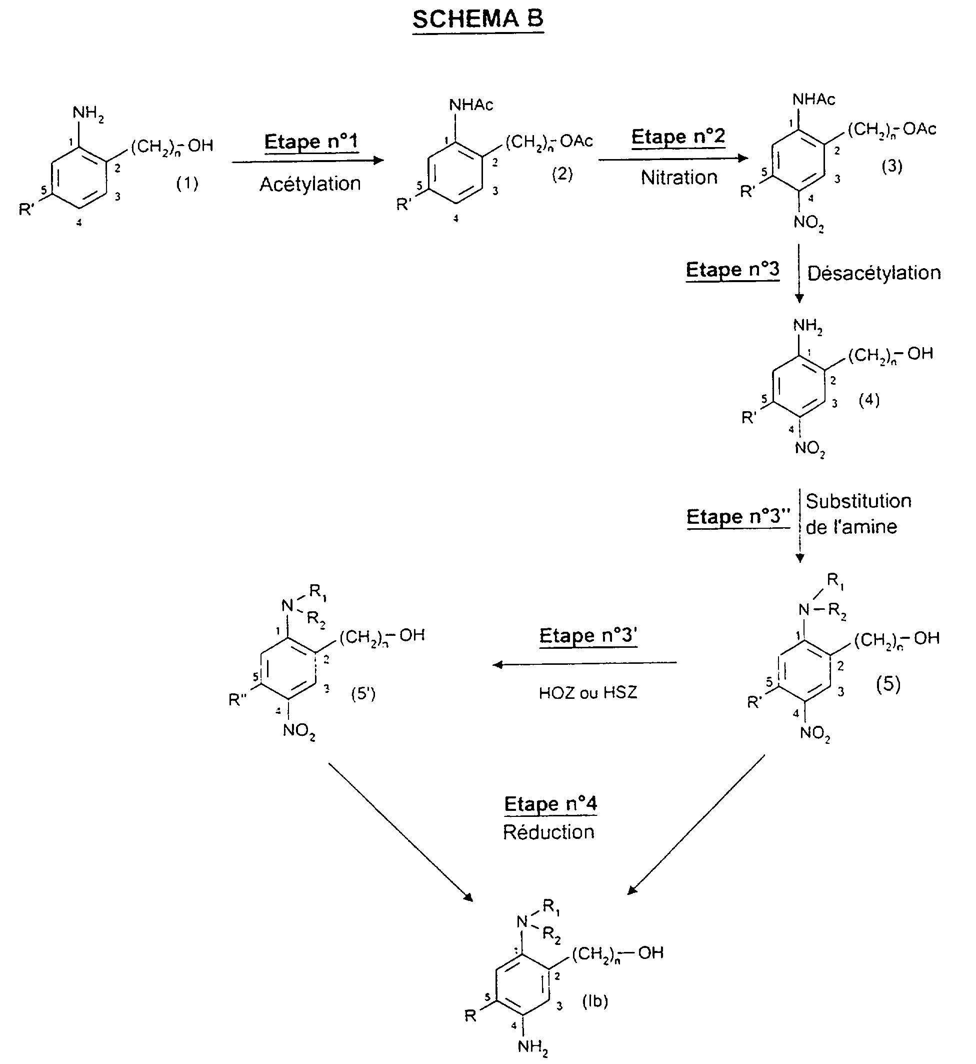

Selon une variante particulière du procédé de synthèse B des composés de formule (Ib) exposé ci-dessus, la substitution du radical R' désignant un atome d'halogène par un radical R" désignant OZ ou SZ (étape 3') peut avoir lieu avant l'étape 3" de substitution de l'amine en position 1. On peut également faire réagir, dans une étape (2'), un composé de formule (3) dans lequel R' désigne un atome d'halogène, avec un alcool de formule HOZ ou un thiol de formule HSZ, Z ayant les mêmes significations que celles indiquées pour la formule (1), pour obtenir un composés de formule (3') telle que définie ci-dessus, puis effectuer une réaction de désacétylation pour obtenir un composé de formule (4') telle que définie précédemment, suivie de la substitution de la fonction amine en position 1 du noyau benzénique par un radical R₁ et/ou R₂, pour obtenir un composé de formule (5').

Cette variante particulière du procédé de synthèse B peut être représentée par le schéma de synthèse B' ci-après :

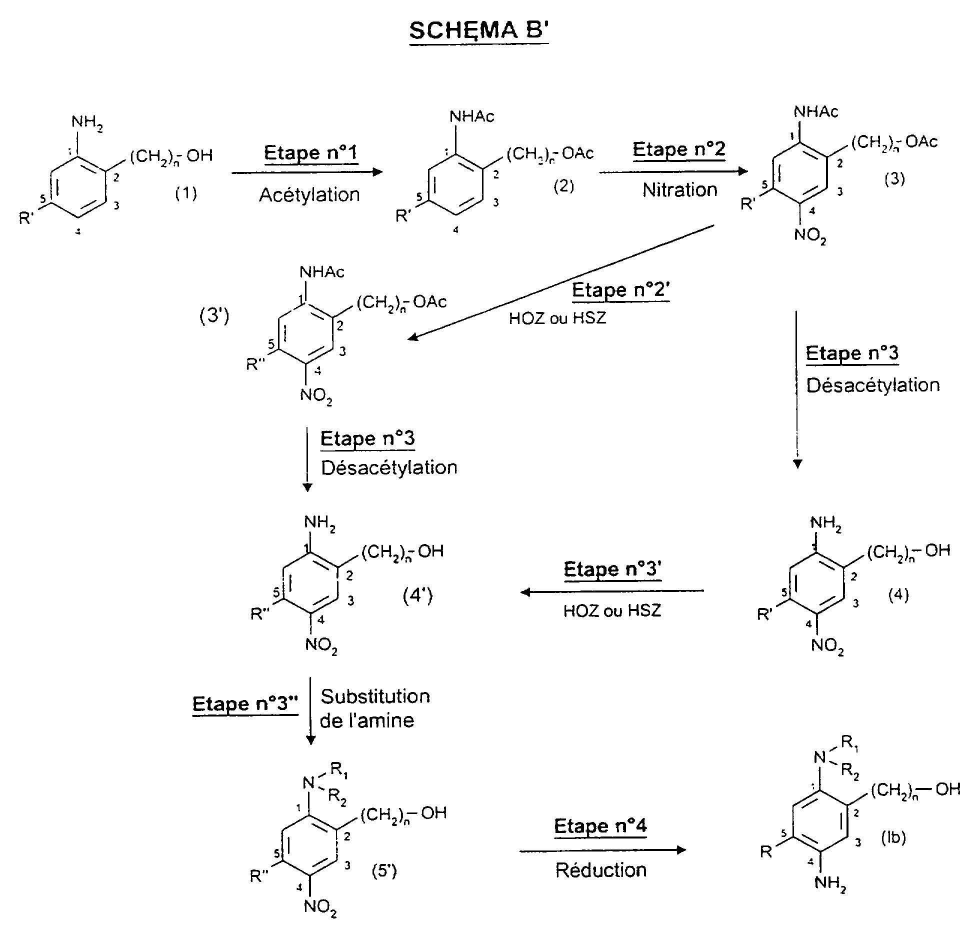

Selon une troisième forme de réalisation du procédé de synthèse conforme à l'invention, les composés particuliers de formule (Ic) suivante et leurs sels d'addition avec un acide 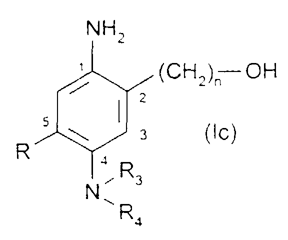 dans laquelle R, R₃, R₄ et n ont les mêmes significations que celles indiquées pour la formule (I) ci-dessus, étant entendu qu'au moins un des radicaux R₃ et R₄ est différent d'un atome d'hydrogène, peuvent être préparés selon le procédé de synthèse C consistant à réaliser les étapes 1 et 2 du procédé de synthèse A décrit précédemment pour obtenir un composé de formule (3) telle que décrite ci-dessus, puis pour obtenir les composés de formule (Ic) dans lesquels R désigne un groupe OZ ou SZ, à faire réagir dans une étape 3' un composé de formule (3), dans lequel R' désigne un atome d'halogène, avec un alcool de formule HOZ ou un thiol de formule HSZ dans lesquelles Z a les mêmes significations que celles indiquées pour la formule (Ic), pour obtenir un composé de formule (3') suivante : 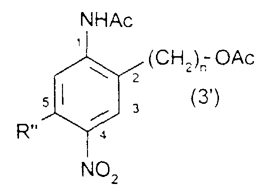 dans laquelle R" et Ac ont les mêmes significations que celles indiquées précédemment, puis dans une quatrième étape, à effectuer une réaction de réduction sur le composé de formule (3) ou (3'), étant entendu que lorsqu'on fait réagir le composé de formule (3) dans laquelle R' désigne un atome d'halogène, le mode de réduction est choisi parmi les procédés suivants :
- pour obtenir les composés de formule (Ic) dans laquelle R désigne un atome d'hydrogène, on effectue une réaction de réduction déshalogénante ;
- pour obtenir les composés de formule (Ic) dans laquelle R désigne un atome d'halogène, on effectue une réaction de réduction non déshalogénante ;
   pour obtenir un composé de formule (6) suivante : 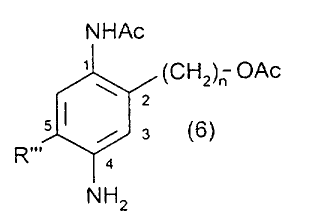 dans laquelle Ac a la même signification que celle indiquée précédemment et R"' représente un radical R' ou R", R' et R" ayant les mêmes significations que celles indiquées précédemment, puis dans une cinquième étape à effectuer sur ledit composé de formule (6) la substitution de la fonction amine en position 4 du noyau benzénique par les radicaux R₃ et/ou R₄, pour obtenir un composé de formule (7) suivante : 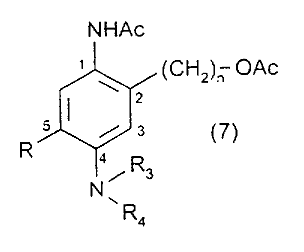
dans laquelle Ac, R₃, R₄, R et n ont les mêmes significations que celles indiquées précédemment, puis dans une sixième étape, à effectuer sur le composé de formule (7), une réaction de désacétylation pour obtenir les composés de formule (Ic) telle que décrite ci-dessus.

Ce procédé de synthèse C peut être représenté par le schéma C suivant :

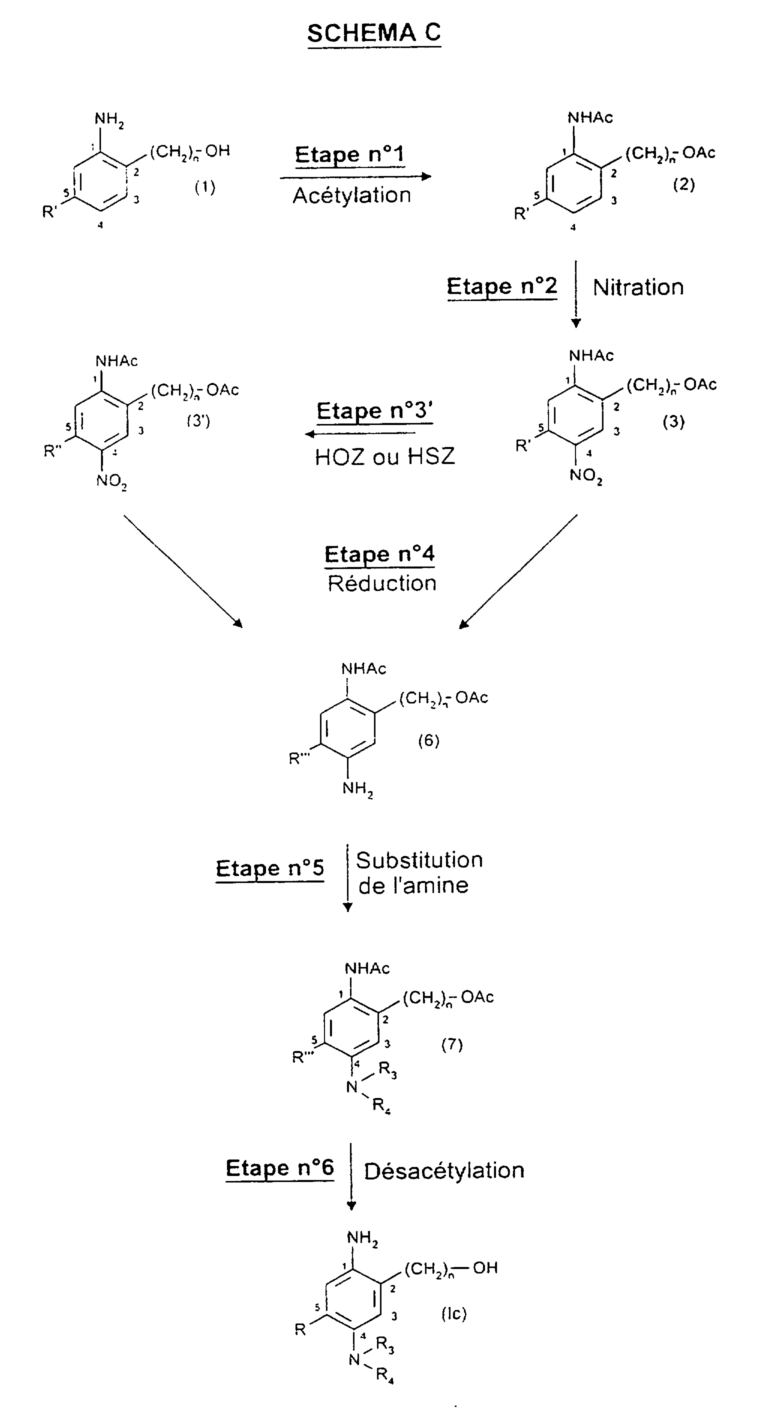

La réaction d'acétylation utilisée lors de la première étape du schéma de synthèse général (Procédés A, B, B' et C) est réalisée selon les méthodes classiques de l'état de la technique telles que par exemple à une température généralement comprise entre 40 et 140°C environ, en présence d'anhydride acétique ou d'un mélange d'acide acétique et d'anhydride acétique.

La réaction de nitration utilisée lors de la deuxième étape se fait selon les méthodes classiques de nitration connues de l'état de la technique, de préférence dans l'acide sulfurique, à une température généralement comprise entre -20 et +20°C, l'agent nitrant étant de préférence de l'acide nitrique dans de l'acide sulfurique (mélange sulfonitrique).

La réaction de désacétylation utilisée lors de la troisième étape se fait selon les méthodes classiquement utilisées dans la littérature, de préférence en milieu acide tel que par exemple en présence d'acide chlorhydrique, et à une température généralement comprise entre 20 et 100°C environ.

La réaction de réduction utilisée lors de la quatrième étape se fait par les procédés bien connus de la littérature, de préférence dans l'alcool, tel que par exemple dans l'éthanol, à une température généralement comprise entre 20 et 100°C.

Lorsque l'on veut obtenir un composé de formule (I) non substitué en position 5 du noyau benzénique (R = H), on effectue, sur un composé de formule (4) pour le procédé de synthèse A ou sur un composé de formule (5) pour le procédé de synthèse B, ou de formule (3) pour le procédé de synthèse C, dans lesquels R' représente un atome d'halogène, une réaction de réduction déshalogénante par réduction catalytique en présence d'un catalyseur tel que par exemple le palladium sur charbon :
- soit sous une pression d'hydrogène généralement comprise entre 1 et 50 bars ;
- soit en présence d'un donneur d'hydrogène tel que par exemple le cyclohexène, l'acide formique ou le formiate d'ammonium.

Lorsque l'on veut obtenir un composé de formule (I) substitué en position 5 du noyau benzénique par un atome d'halogène (R = halogène), on effectue une réduction non déshalogénante sur un composé de formule (4) pour le procédé de synthèse A ou sur un composé de formule (5) pour le procédé de synthèse B, ou de formule (3) pour le procédé de synthèse C, dans lesquels R' représente un atome d'halogène par exemple par réduction dans un milieu Fer/acétique ou par réduction dans un mélange Zinc/eau/alcool.

Lorsque l'on veut obtenir un composé de formule (I) dans lequel R est différent d'un atome d'hydrogène ou d'halogène, tout mode de réduction classiquement utilisé dans l'état de la technique peut être employé.

Toutes les conditions de réactions décrites ci-dessus sont largement décrites dans la littérature, ne sont que des préférences et des exemples, et ne limitent aucunement les différentes conditions opératoires pouvant être utilisées selon le procédé de synthèse conforme à l'invention.

Certaines 2-hydroxyalkyl paraphénylènediamines de formule (I) définie ci-dessus sont nouvelles et constituent à ce titre un autre objet de l'invention.

Ces nouvelles 2-hydroxyalkyl paraphénylènediamines et leurs sels d'addition avec un acide répondent à la formule (I') suivante : 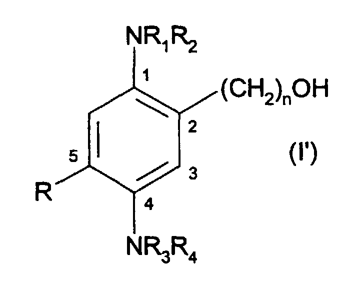 dans laquelle :
- n est un nombre entier compris entre 1 et 4 inclusivement ;
- R₁, R₂, R₃, R₄ , identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle; carboxyalkyle en C₁-C₆ ; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
- R désigne un atome d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ; étant entendu
- qu'au moins un des groupes -NR₁R₂ et -NR₃R₄ désigne un groupement -NH₂ ;
- que lorsque R₁ et R₂ représentent simultanément un atome d'hydrogène, et que n est égal à 1, alors R est différent d'un radical méthyle ou d'un radical éthyle.

Les réserves figurant ci-dessus ont été introduites pour exclure de l'invention des composés connus de l'état de la technique, notamment dans le domaine photographique, (US 2 273 564 ; TONG L K J et Al, Journal of The American Chemical Society, Vol. 82, n° 8, 25 avril 1960, pages 1988-1996 ; R. L. BENT, Journal of The American Chemical Society, Vol. 73, 1951, DC US, pages 3100-3125).

Parmi les 2-hydroxyalkyl paraphénylènediamines de formule (I') ci-dessus on peut notamment citer :
- la 2-(β-hydroxyéthyl) 5-chloro paraphénylènediamine,
- la 2-(β-hydroxyéthyl) 5-méthoxy paraphénylènediamine,
- la 2-(β-hydroxyéthyl) 5-méthylthio paraphénylènediamine,
- la 2-(β-hydroxyéthyl) 5-méthyl paraphénylènediamine,
et leurs sels d'addition avec un acide.

Les nouvelles 2-hydroxyalkyl paraphénylènediamines de formule (I') conformes à l'invention peuvent être utilisées à titre de base d'oxydation dans des compositions pour la teinture d'oxydation des fibres kératiniques et en particulier des fibres kératiniques humaines telles que les cheveux, ce qui constitue un autre objet de l'invention.

L'invention a également pour objet la composition tinctoriale pour la teinture des fibres kératiniques et en particulier des fibres kératiniques humaines telles que les cheveux, caractérisée par le fait qu'elle contient, dans un milieu approprié pour la teinture, au moins une 2-hydroxyalkyl paraphénylènediamine de formule (I') telle que définie précédemment, à titre de base d'oxydation.

La ou les 2-hydroxyalkyl paraphénylènediamine de formule (I') conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (Il) suivante 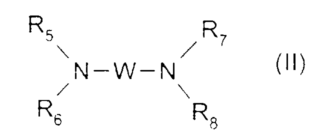 dans laquelle W est un reste propylène éventuellement substitué par un groupement hydroxyle ou un radical alkyle en C₁-C₆ ; R₅, R₆, R₇ et R₈, identiques ou différents, représentent un atome d'hydrogène, un radical alkyle en C₁-C₆ ou hydroxyalkyle en C₁-C₆.

La composition tinctoriale conforme à l'invention peut encore contenir, en plus des 2-hydroxyalkyl paraphénylènediamine de formule (I') définie ci-dessus, au moins une base d'oxydation additionnelle qui peut être choisie parmi les bases d'oxydation classiquement utilisées en teinture d'oxydation et parmi lesquelles on peut notamment citer les paraphénylènediamines différentes des composés de formule (I') conformes à l'invention, les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple. le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formules (I), (Ia), (Ib), (Ic), (I'), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatile ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les peroxydases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLE 1 : Préparation du dichlorhydrate de 5-chloro 2-β-hydroxyéthyl paraphénylènediamine

a) Synthèse de l'acétate de 2-(2-acétylamino-4-chloro-5-nitro-phényl)-éthyle
Dans un réacteur, on a fait le mélange de 76,0 g (0,442 mole) de 1-amino 5-chloro 2-β-hydroxyéthyl benzène dans 30 ml d'acide acétique et 100 ml d'anhydride acétique. La réaction était exothermique. On a chauffé au bain-marie bouillant sous agitation pendant 2 heures jusqu'à disparition (en Chromatographie sur Couche Mince (CCM) du N-[5-Chloro-2-(2-hydroxyéthyl)-phényl]-acétamide intermédiaire.
Après refroidissement dans un bain de glace (prise en masse), on a essoré, lavé à l'éther de pétrole et séché sous vide et sur potasse les cristaux blancs (73,6 g) d'acétate de 2-(2-acétylamino-4-chlorophényl)-éthyle qui ont fondu à 129°C (Kofler).
Dans un réacteur on a placé 215 ml d'acide sulfurique 98% et refroidi à 0°C.
Sous agitation on a ajouté par portions les 73,6 g (0,287 mole) du composé obtenu ci-dessus en maintenant la température à 0°C (dissolution lente).
Puis on a coulé goutte à goutte, en 30 minutes et en maintenant la température entre - 5°C et 0°C, une solution de 20,6 ml d'acide nitrique (d=1,50) dans 110 ml d'acide sulfurique à 98%.
On a laissé l'agitation pendant 30 minutes supplémentaires entre -5°C et 0°C et coulé le milieu réactionnel dans 1,6 kg d'eau glacée.
Après avoir essoré le précipité, réempaté plusieurs fois dans l'eau, et séché sous vide à 40°C sur anhydride phosphorique, on a obtenu des cristaux beiges (73,7 g) d'acétate de 2-(2-acétylamino-4-chloro-5-nitro-phényl)-éthyle qui ont fondu à 153°C (Kofler) et dont l'analyse élémentaire calculée pour C₁₂H₁₃N₂O₅Cl était :

| % | C | H | N | O | CI |
|---|---|---|---|---|---|
| Calculé | 47,93 | 4,36 | 9,32 | 26,60 | 11,79 |
| Trouvé | 47,96 | 4,39 | 9,15 | 26,61 | 11,64 |

b) Synthèse du 1-amino 5-chloro 2-β-hydroxyéthyl 4-nitro benzène
On a désacétylé 19,7 g (0,065 mole) du produit obtenu ci-dessus à l'étape précédente dans 60 ml d'acide chlorhydrique à 36 % chauffé au bain marie bouillant pendant 45 minutes.
On a coulé sur 200 g d'eau glacée, rendu basique le milieu réactionnel par ajout de lessive de soude 10 N, essoré le précipité, réempaté dans l'eau et séché sous vide à 40°C sur anhydride phosphorique.
On a obtenu 13,6 g de cristaux jaune clair de 1-amino 5-chloro 2-β-hydroxyéthyl 4-nitro benzène qui ont fondu à 176°C (Kofler) et dont l'analyse élémentaire calculée pour C₈H₉N₂O₃Cl était :

| % | C | H | N | O | Cl |
|---|---|---|---|---|---|
| Calculé | 44,36 | 4,19 | 12,93 | 22,16 | 16,37 |
| Trouvé | 44,44 | 4,23 | 12,87 | 22,01 | 16,13 |

c) Réduction du 1-amino 5-chloro 2-β-hydroxyéthyl 4-nitro benzène
On a chauffé au reflux de l'alcool une suspension de 0,4 g de chlorure d'ammonium et 15 g de zinc en poudre fine dans 4 ml d'eau et 23 ml d'éthanol à 96°.
On a ajouté par portions 5,1 g (0,0235 mole) du dérivé nitré obtenu ci-dessus à l'étape précédente, de façon à maintenir le reflux sans chauffage (la réaction étant exothermique). A la fin de l'addition, on a maintenu le reflux pendant 10 minutes supplémentaires, filtré bouillant sur 15 ml d'éthanol chlorhydrique environ 6N glacé et lavé les boues de zinc avec de l'éthanol absolu bouillant.
Le filtrat a été refroidi dans un bain de glace et le précipité essoré et lavé à l'éther éthylique.
Après séchage à 40°C sous vide et sur potasse on a obtenu des cristaux blancs de dichlorhydrate de 5-chloro 2-β-hydroxyéthyl paraphénylènediamine qui ont fondu avec décomposition à 192°-194°C (Kofler) et dont l'analyse élémentaire calculée pour C₈H₁₁N₂OCl,2HCl était :

| % | C | H | N | O | CI |
|---|---|---|---|---|---|
| Calculé | 37,02 | 5,05 | 10,79 | 6,16 | 40,98 |
| Trouvé | 37,03 | 5,09 | 10,59 | 6,77 | 40,44 |

### EXEMPLE 2 : Préparation de la 2-β-hydroxyéthyl 5-méthoxy paraphénylènediamine, dichlorhydrate, ¼ H₂O

a) Synthèse du 1-amino 2-β-hydroxyéthyl 5-méthoxy 4-nitro benzène
On a chauffé pendant 4 heures au reflux, le mélange de 21,6 g (0,1 mole) de 1-amino 5-chloro 2-β-hydroxyéthyl 4-nitro benzène obtenu ci-dessus à l'exemple 1, étape b), de 50 ml de méthanol et de 40 ml d'une solution de méthylate de sodium à 30% dans le méthanol.
On a versé sur 500 ml d'eau glacée, essoré le précipité cristallisé, réempaté dans l'eau et séché sous vide à 40°C sur anhydride phosphorique.
On a obtenu des cristaux jaunes (15,8 g) qui, après purification par recristallisation de l'éthanol bouillant, ont fondu à 145°C (Kofler) et dont l'analyse élémentaire calculée pour C₉H₁₂N₂O₄ était :

| % | C | H | N | O |
|---|---|---|---|---|
| Calculé | 50,94 | 5,70 | 13,20 | 30,16 |
| Trouvé | 50,85 | 5,71 | 13,16 | 30,22 |

b) Réduction du 1-amino 2-β-hydroxyéthyl 5-méthoxy 4-nitro benzène
On a utilisé le mode opératoire décrit pour l'exemple 1, étape c).
Par réduction de 10,0 g (0,047 mole) de 1-amino 2-β-hydroxyéthyl 5-méthoxy 4-nitro benzène obtenu à l'étape précédente, on a obtenu 10,7 g de cristaux blancs de dichlorhydrate de 2-β-hydroxyéthyl 5-méthoxy paraphénylènediamine (cristallisés avec ¼ H₂0) qui ont fondu avec décomposition à 216-218°C (Kofler) et dont l'analyse élémentaire calculée pour C₉H₁₄N₂O₂,2HCl + ¼ H₂O était :

| % | C | H | N | O | CI |
|---|---|---|---|---|---|
| Calculé | 41,63 | 6,41 | 10,79 | 13,86 | 27,31 |
| Trouvé | 41,58 | 6,57 | 10,70 | 14,50 | 27,31 |

### EXEMPLE 3 : Préparation de la 2-β-hydroxyéthyl 5-méthylthio paraphénylènediamine, dichlorhydrate

a) Synthèse du 1-amino 2-β-hydroxyéthyl 5-méthylthio 4-nitro benzène
On a fait une suspension de 9,5 g de thiométhylate de sodium dans 100 ml de 1,2-diméthoxyéthane et sous agitation on a ajouté 21,6 g (0,1 mole) de 1-amino 5-chloro 2-β-hydroxyéthyl 4-nitro benzène obtenu ci-dessus à l'exemple 1, étape b). On a chauffé 30 minutes à 50°C puis 10 minutes à 65°C.
On a versé sur 800 ml d'eau glacée, essoré le précipité cristallisé, réempaté dans l'eau et séché sous vide à 40°C sur anhydride phosphorique.
On a obtenu des cristaux jaunes (21,0 g) qui, après purification par recristallisation de l'acétate d'éthyle bouillant, ont fondu à 180°C (Kofler) et dont l'analyse élémentaire calculée pour C₉H₁₂N₂O₃S était :

| % | C | H | N | O | S |
|---|---|---|---|---|---|
| Calculé | 47,36 | 5,30 | 12,27 | 21,03 | 14,05 |
| Trouvé | 47,92 | 5,27 | 12,05 | 21,49 | 13,38 |

b) Réduction du 1-amino 2-β-hydroxyéthyl 5-méthylthio 4-nitro benzène
On a utilisé le mode opératoire décrit ci-dessus pour l'exemple 1, étape c).
Par réduction de 15,5 g (0,068 mole) de 1-amino 2-β-hydroxyéthyl 5-méthylthio 4-nitro benzène obtenu précédemment à l'étape a), on a obtenu 16,8 g de cristaux blancs de dichlorhydrate de 2-β-hydroxyéthyl 5-méthylthio paraphénylènediamine qui ont fondu avec décomposition à 182-184°C (Kofler) et dont l'analyse RMN ¹H était conforme à celle du produit attendu.

### EXEMPLE 4 : Préparation de la 2-β-hydroxyéthyl 5-méthyl paraphénylènediamine, dichlorhydrate

a) Synthèse de l'acétate de 2-(2-acétylamino-4-méthyl-5-nitro-phényl)-éthyle
On a utilisé le mode opératoire décrit ci-dessus pour l'exemple 1, étape a).
En partant de 23,5 g (0,155 mole) de 1-amino 2-β-hydroxyéthyl 5-méthyl benzène, on a obtenu des cristaux crème (19,2 g) d'acétate de 2-(2-acétylamino-4-méthyl-5-nitro-phényl)-éthyle qui, après purification par recristallisation de l'acétate d'éthyle bouillant, ont fondu à 125°C (Kofler) et dont l'analyse élémentaire calculée pour C₁₃H₁₆N₂O₅, ½ H₂O était :

| % | C | H | N | O |
|---|---|---|---|---|
| Calculé | 53,98 | 5,92 | 9,68 | 30,42 |
| Trouvé | 54,24 | 5,68 | 9,70 | 30,42 |

b) Désacétylation - Réduction de l'acétate de 2-(2-acétylamino-4-méthyl-5-nitrophényl)-éthyle
Par désacétylation, (selon le mode opératoire décrit ci-dessus pour l'étape b de l'exemple 1), de 19,0 g (0,067 mole) du composé obtenu ci-dessus à l'étape précédente, on a obtenu, après purification par recristallisation de l'éthanol bouillant, 7,5 g de cristaux jaune pâle de 1-amino 2-β-hydroxyéthyl 5-méthyl 4-nitro benzène dont le point de fusion (Kofler) était de 168°C.
La réduction selon le mode opératoire décrit ci-dessus pour l'exemple 1, étape c) de ces 7,5 g (0,038 mole) de 1-amino 2-β-hydroxyéthyl 5-méthyl 4-nitro benzène a donné des cristaux blancs (7,5 g) de dichlorhydrate de 2-β-hydroxyéthyl 5-méthyl paraphénylènediamine qui ont fondu avec décomposition à 208-210°C (Kofler) et dont l'analyse élémentaire calculée pour C₉H₁₆N₂OCl₂ était

| % | C | H | N | O | CI |
|---|---|---|---|---|---|
| Calculé | 45,20 | 6,74 | 11,71 | 6,69 | 29,65 |
| Trouvé | 45,11 | 6,77 | 11,65 | 7,09 | 29,47 |

### EXEMPLE 5 : Préparation du 4-amino 2-β-hydroxyéthyl 1-N-(β-hydroxyéthyl)-amino benzène, dichlorhydrate

a) Synthèse du 5-chloro 1-N-(β-hydroxyéthyl)amino 2-β-hydroxyéthyl 4-nitro benzène
On a chauffé au bain-marie bouillant le mélange de 10,8 g (0,05 mole) de 1-amino 5-chloro 2-β-hydroxyéthyl 4-nitro benzène obtenu ci-dessus à l'exemple 1 étape b) et de 5,5 g (0,055 mole) de carbonate de calcium dans 50 ml de dioxane.
On a ajouté goutte à goutte 5,7 ml (0,055 mole) de β-chloréthylchloroformiate et prolongé le chauffage pendant une heure.
On a versé sur 300 ml d'eau glacée, acidifié avec de l'acide chlorhydrique, essoré le précipité blanc cristallisé, réempaté dans l'eau et mis en suspension dans 27 ml d'eau.
On a ajouté 18 ml de lessive de soude 10N et chauffé à 65°C pendant une heure sous agitation.
Une huile a décanté et cristallisé. On a essoré, réempaté dans l'eau et séché sous vide.
On a obtenu des cristaux jaunes (10,9 g) qui, après purification par recristallisation de l'éthanol bouillant, ont fondu à 129°C (Kofler) et dont l'analyse élémentaire calculée pour C₁₀H₁₃N₂O₄Cl était :

| % | C | H | N | O | CI |
|---|---|---|---|---|---|
| Calculé | 46,08 | 5,03 | 24,55 | 10,75 | 13,60 |
| Trouvé | 45,78 | 5,02 | 24,46 | 10,63 | 13,42 |

b) Réduction déshalogénante du 5-chloro 1-N-(β-hydroxyéthyl)amino 2-β-hydroxyéthyl 4-nitro benzène
On a chauffé au reflux un mélange de 8,0 g (0,0306 mole) de 5-chloro 1-N-(β-hydroxyéthyl)amino 2-β-hydroxyéthyl 4-nitro benzène obtenu ci-dessus à l'étape précédente et de 9,0 g de palladium sur charbon à 5% et contenant 50% d'eau dans 30 ml d'éthanol.
On a ajouté goutte à goutte 25 ml de cyclohexène et chauffé au reflux sous agitation pendant 3 heures.
On a filtré bouillant et coulé le filtrat dans 30 ml d'éthanol absolu chlorhydrique 5N.
Après évaporation à sec sous pression réduite et trituration de la gomme obtenue dans un mélange d'éthanol absolu et d'éther éthylique on a obtenu 5,3 g de cristaux blancs hygroscopiques de dichlorhydrate de 4-amino 2-β-hydroxyéthyl 1-N-(β-hydroxyéthyl)amino benzène, qui ont fondu avec décomposition à 120-122°C (Kofler) et dont la structure en RMN ¹H du produit obtenu était conforme à celle du produit attendu.

### EXEMPLES 6 à 11 DE TEINTURE

On a préparé les compositions tinctoriales suivantes (teneurs en grammes) :

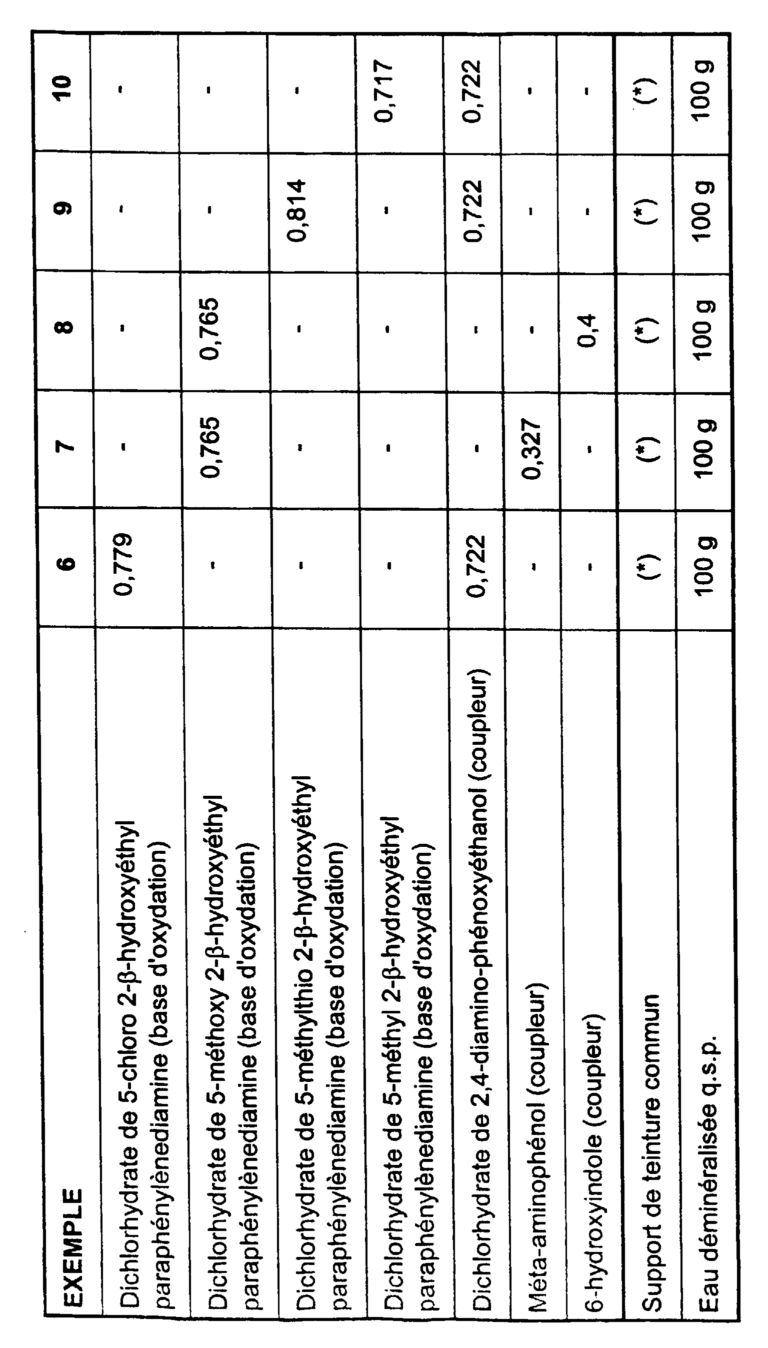

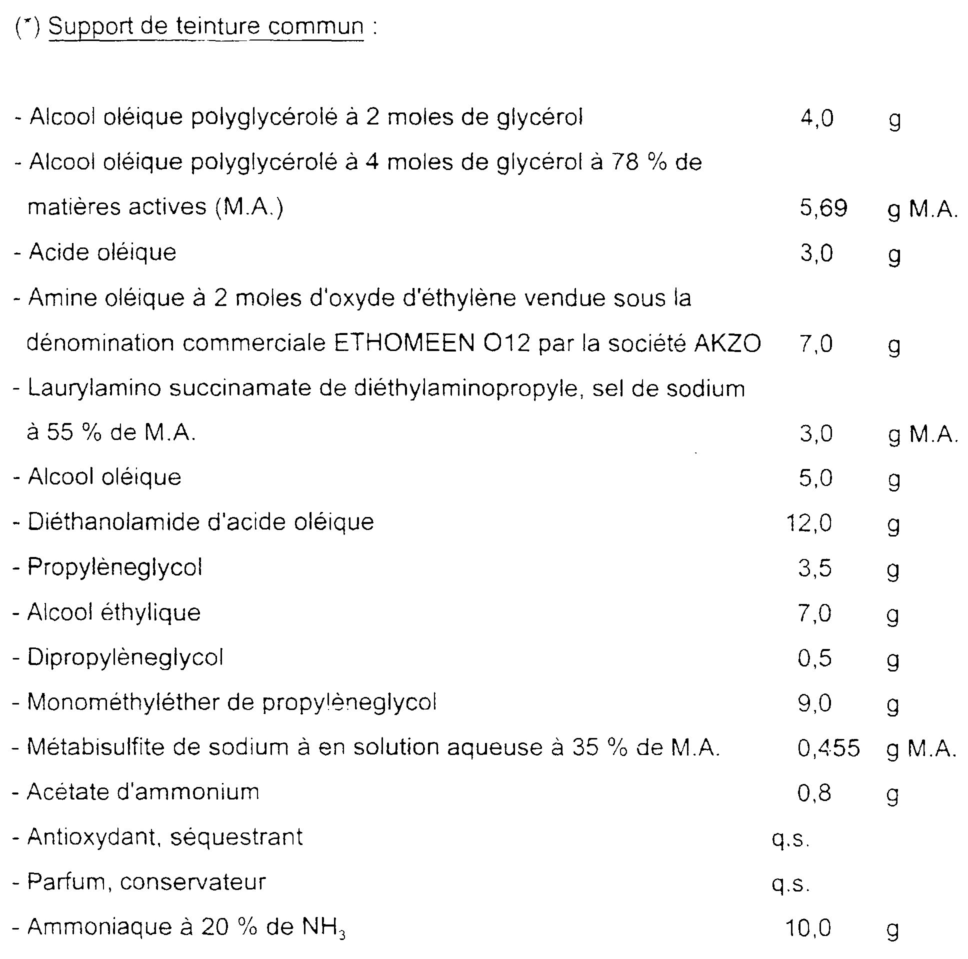

Au moment de l'emploi, on a mélangé chaque composition tinctoriale ci-dessus avec une quantité équivalente en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les nuances obtenues figurent dans le tableau ci-dessous :

| **EXEMPLE** | **pH de TEINTURE** | **NUANCE OBTENUE** |
|---|---|---|
| 6 | 10 ± 0,2 | Blond très clair cendré bleuté |
| 7 | 10 ± 0,2 | Blond cendré très bleu |
| 8 | 10 ± 0,2 | Blond beige acajou |
| 9 | 10 ± 0,2 | Blond clair cendré bleuté |
| 10 | 10 ± 0,2 | Blond bleu cendré |

## Revendications

1. Procédé de synthèse de 2-hydroxyalkyl paraphénylènediamines substituées ou non substituées en position 5 du noyau benzénique et de leurs sels d'addition avec un acide, caractérisé par le fait qu'il comprend au moins les étapes suivantes :
Etape n°1 : on protège la fonction amine en position 1 du noyau benzénique et la fonction alcool en position 2 du noyau benzénique d'une 2-hydroxyalkyle aniline substituée en position 5 du noyau benzénique par un atome d'halogène ou par un radical alkyle en C₁-C₆ ;
Etape n°2 : on effectue, sur le composé obtenu à l'étape n° 1, une réaction de nitration en position 4 du noyau benzénique ;
Etape n°3 : on effectue la déprotection de la fonction amine en position 1 du noyau benzénique et de la fonction alcool en position 2 du noyau benzénique du composé obtenu à l'étape n° 2 :
Etape n°4 : on effectue sur le composé obtenu à l'étape n° 3, une réaction de réduction du groupe nitro en position 4, accompagnée ou non, dans le cas d'un composé 5-halogéné, d'une déshalogénation.

2. Procédé selon la revendication 1, caractérisé par le fait que les 2-hydroxyalkyl paraphénylènediamines substitués ou non substituées en position 5 sur le noyau benzénique, ainsi que leurs sels d'addition avec un acide répondent à la formule (I) suivante : 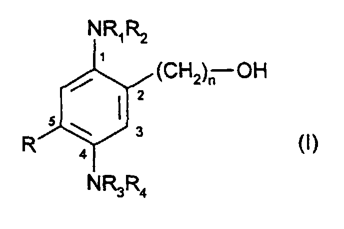 dans laquelle :
- n est un nombre entier compris entre 1 et 4 inclusivement ;
- R désigne un atome d'hydrogène ou d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ;
- R₁, R₂, R₃, R₄, identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle ; carboxyalkyle en C₁-C₆ ; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
étant entendu qu'au moins un des groupes -NR₁R₂ et -NR₃R₄ désigne un groupement -NH₂.

3. Procédé de synthèse selon la revendication 1 ou 2 des composés répondant à la formule (Ia) suivante, et de leurs sels d'addition avec un acide : 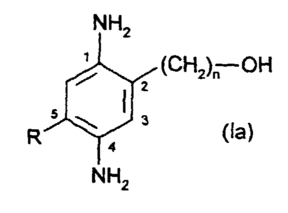 dans lesquels :
- R₁, R₂, R₃ et R₄ désignent simultanément un atome d'hydrogène ;
- R désigne un atome d'hydrogène ou d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ;
- n est un nombre entier compris entre 1 et 4 inclusivement ;
ledit procédé comprenant les étapes suivantes :
(a) on effectue sur un composé de formule (1) suivante : 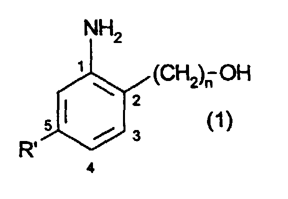 dans laquelle R' désigne un atome d'halogène ou un radical alkyle en C₁-C₆, et n est un nombre entier compris entre 1 et 4 inclusivement, une réaction d'acétylation pour obtenir un composé de formule (2) suivante : 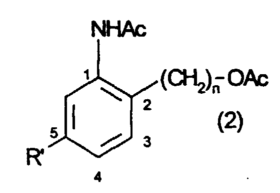 dans laquelle Ac désigne le groupe acétyle, R' et n ont les mêmes significations que celles indiquées ci-dessus pour la formule (1) ;
(b) on effectue sur le composé de formule (2) une réaction de nitration en position 4 du noyau benzénique, pour obtenir un composé de formule (3) suivante : 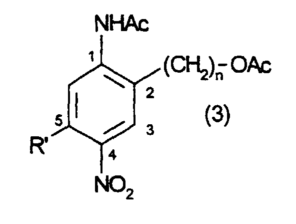 dans laquelle R', n et Ac ont les mêmes significations que celles indiquées ci-dessus pour la formule (2) ;
(c) on effectue sur le composé de formule (3) une réaction de désacétylation pour obtenir le composé de formule (4) suivante : 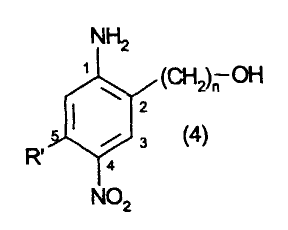 dans laquelle R' et n ont les mêmes significations que celles indiquées ci-dessus pour la formule (3) ; puis
(d) pour obtenir les composés de formule (la) dans lesquels R désigne un groupe OZ ou SZ, on fait réagir le composé de formule (4), dans lequel R' désigne un atome d'halogène, avec un alcool de formule HOZ ou un thiol de formule HSZ dans lesquelles Z a la même signification que celle indiquée pour la formule (la) pour obtenir un composé de formule (4') suivante : 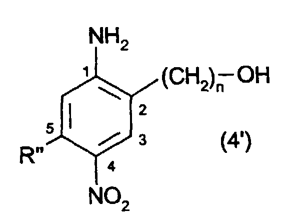 dans laquelle R" désigne un groupe OZ ou SZ, Z et n ayant les mêmes significations que celles indiquées ci-dessus pour la formule (Ia) ;
(e) on effectue une réaction de réduction sur le composé de formule (4) ou de formule (4'),
étant entendu que lorsqu'on fait réagir le composé de formule (4) dans lequel R' désigne un atome d'halogène, le mode de réduction est choisi parmi les procédés suivants :
- pour obtenir les composés de formule (la) dans laquelle R désigne un atome d'hydrogène, on effectue une réaction de réduction déshalogénante ;
- pour obtenir les composés de formule (la) dans laquelle R désigne un atome d'halogène, on effectue une réaction de réduction non déshalogénante.

4. Procédé de synthèse selon la revendication 1 ou 2 des composés répondant à la formule (Ib) suivante, et de leurs sels d'addition avec un acide : 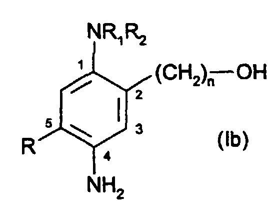 dans laquelle :
- R désigne un atome d'hydrogène ou d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ;
- R₁ et R₂, identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle; carboxyalkyle en C₁-C₆ ; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
- n est un nombre entier compris entre 1 et 4 inclusivement ;
étant entendu qu'au moins un des radicaux R₁ et R₂ est différent d'un atome d'hydrogène ;
ledit procédé comprenant les étapes suivantes :
(a) on effectue sur un composé de formule (1) suivante : 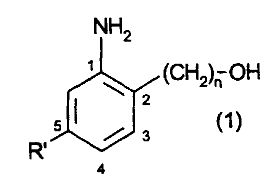 dans laquelle R' désigne un atome d'halogène ou un radical alkyle en C₁-C₆, et n est un nombre entier compris entre 1 et 4 inclusivement, une réaction d'acétylation pour obtenir un composé de formule (2) suivante : 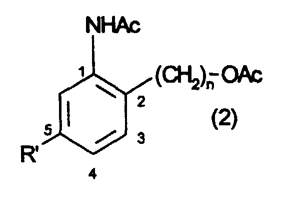 dans laquelle Ac désigne le groupe acétyle, R' et n ont les mêmes significations que celles indiquées ci-dessus pour la formule (1) ;
(b) on effectue sur le composé de formule (2) une réaction de nitration en position 4 du noyau benzénique, pour obtenir un composé de formule (3) suivante : 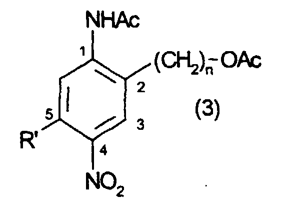 dans laquelle, R', n et Ac ont les mêmes significations que celles indiquées ci-dessus pour la formule (2) ;
(c) on effectue sur le composé de formule (3) une réaction de désacétylation pour obtenir le composé de formule (4) suivante : 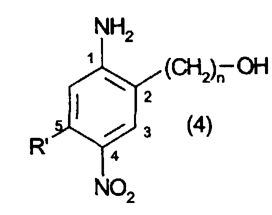 dans laquelle R' et n ont les mêmes significations que celles indiquées ci-dessus pour la formule (3) ;
(d) on effectue sur le composé de formule (4) la substitution de la fonction amine en position 1 du noyau benzénique par les radicaux R₁ et/ou R₂, pour obtenir un composé de formule (5) suivante : 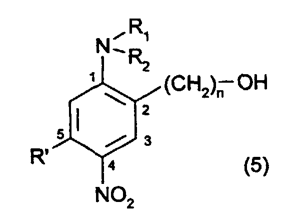 dans laquelle R₁, R₂ et n ont les mêmes significations que celles indiquées ci-dessus pour la formule (Ib) et R' a la même signification que celle indiquée ci-dessus pour la formule (1) ; puis
(e) pour obtenir les composés de formule (Ib) dans lesquels R désigne un groupe OZ ou SZ, on fait réagir le composé de formule (5) dans laquelle R' désigne un atome d'halogène, avec un alcool de formule HOZ ou un thiol de formule HSZ dans lesquelles Z a la même signification que celles indiquées ci-dessus pour la formule (Ib), pour obtenir un composé de formule (5') suivante : 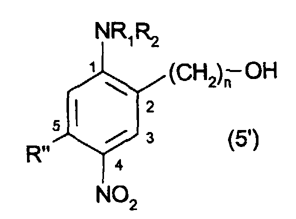 dans laquelle R" désigne un groupe OZ ou SZ dans lesquels Z et n ont les mêmes significations que celles indiquées pour la formule (Ib) ci-dessus ;
(f) on effectue une réaction de réduction sur le composé de formule (5) ou de formule (5'), étant entendu que lorsqu'on fait réagir un composé de formule (5) dans laquelle R' désigne un atome d'halogène, le mode de réduction est choisi parmi les procédés suivants :
- pour obtenir les composés de formule (Ib) dans laquelle R désigne un atome d'hydrogène, on effectue une réaction de réduction déshalogénante ;
- pour obtenir les composés de formule (Ib) dans laquelle R désigne un atome d'halogène, on effectue une réaction de réduction non déshalogénante.

5. Procédé de synthèse selon la revendication 1 ou 2 des composés répondant à la formule (Ic) suivante, et de leurs sels d'addition avec un acide : 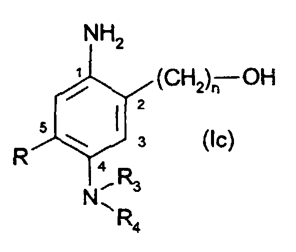 dans laquelle :
- R désigne un atome d'hydrogène ou d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ;
- R₃ et R₄, identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle; carboxyalkyle en C₁-C₆ ; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
- n est un nombre entier compris entre 1 et 4 inclusivement ;
étant entendu qu'au moins un des radicaux R₃ et R₄ est différent d'un atome d'hydrogène ;
ledit procédé comprenant les étapes suivantes :
(a) on effectue sur un composé de formule (1) suivante : 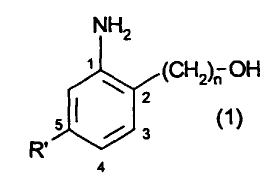 dans laquelle R' désigne un atome d'halogène ou un radical alkyle en C₁-C₆, et n est un nombre entier compris entre 1 et 4 inclusivement, une réaction d'acétylation pour obtenir un composé de formule (2) suivante : 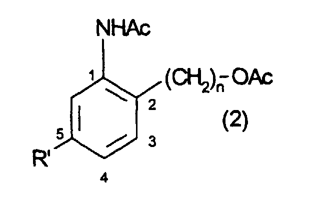 dans laquelle Ac désigne le groupe acétyle, R' et n ont les mêmes significations que celles indiquées ci-dessus pour la formule (1) ;
(b) on effectue sur le composé de formule (2) une réaction de nitration en position 4 du noyau benzénique, pour obtenir un composé de formule (3) suivante : 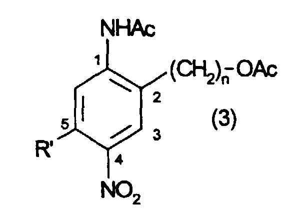 dans laquelle, R', n et Ac ont les mêmes significations que celles indiquées ci-dessus pour la formule (2) ;
(c) pour obtenir les composés de formule (Ic) dans lesquels R désigne un groupe OZ ou SZ, Z ayant les mêmes significations que celles indiquées ci-dessus pour les composés de formule (Ic), on fait réagir le composé de formule (3) dans laquelle R' désigne un atome d'halogène avec un alcool de formule HOZ ou un thiol de formule HSZ dans lesquelles Z a les mêmes significations que celles indiquées ci-dessus pour les composés de formule (Ic) pour obtenir un composé de formule (3') suivante : 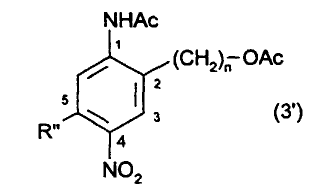 dans laquelle Ac représente le groupe acétyle, R" désigne un groupe OZ ou SZ, Z et n ayant les mêmes significations que celles indiquées ci-dessus pour les composés de formule (Ic) ;
(d) on effectue une réaction de réduction sur le composé de formule (3) ou de formule (3'), étant entendu que lorsqu'on fait réagir le composé de formule (3) dans laquelle R' désigne un atome d'halogène, le mode de réduction est choisi parmi les procédés suivants :
- pour obtenir les composés de formule (Ic) dans laquelle R désigne un atome d'hydrogène, on effectue une réaction de réduction déshalogénante ;
- pour obtenir les composés de formule (Ic) dans laquelle R désigne un atome d'halogène, on effectue une réaction de réduction non déshalogénante ;
pour obtenir un composé de formule (6) suivante : 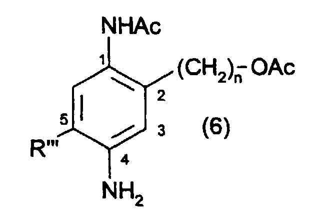 dans laquelle R"' désigne R' ou R", R' et R" ayant les mêmes significations que celles indiquées ci-dessus pour les composés de formules (3) et (3'), Ac représente le groupe acétyle et n ayant les mêmes significations que celles indiquées ci-dessus pour les composés de formule (Ic) ;
(e) on effectue sur le composé de formule (6) la substitution de la fonction amine en position 4 du noyau benzénique par les radicaux R₃ et/ou R₄, pour obtenir un composé de formule (7) suivante : 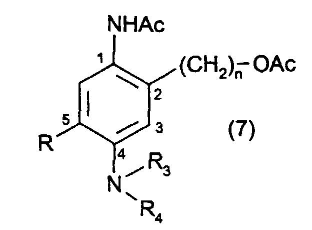 dans laquelle R, R₃, R₄, Ac et n ont les mêmes significations que celles indiquées ci-dessus pour les formules (Ic) et (2) ;
(f) on effectue sur le composé de formule (7) une réaction de désacétylation pour obtenir le composé de formule (Ic).

6. Procédé selon l'une quelconque des revendications précédentes, caractérisé par le fait que les sels d'addition avec un acide sont choisis parmi les chlorhydrates, les bromhydrates, les sulfates, les citrates, les succinates, les tartrates, les lactates et les acétates.

7. 2-hydroxyalkyl paraphénylènediamines répondant à la formule (I') suivante, et leurs sels d'addition avec un acide : 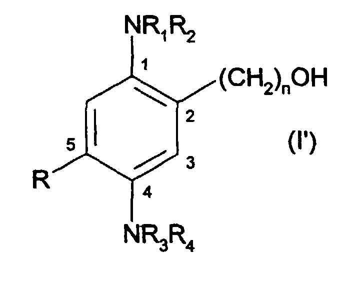 dans laquelle :
- n est un nombre entier compris entre 1 et 4 inclusivement ;
- R₁, R₂, R₃, R₄ , identiques ou différents, désignent un atome d'hydrogène, un radical alkyle en C₁-C₆, monohydroxyalkyle en C₁-C₆, polyhydroxyalkyle en C₂-C₆, alcoxy(C₁-C₆)alkyle en C₁-C₆, aminoalkyle en C₁-C₆, mono- ou dihydroxyalkyl(C₁-C₆)aminoalkyle en C₁-C₆ ; mono- ou dialkyl(C₁-C₆)aminoalkyle en C₁-C₆ ; aminoalkyle dont l'amine est protégée par un radical alkyl(C₁-C₆)carbonyle, carbamyle ou alkyl(C₁-C₆)sulfonyle; carboxyalkyle en C₁-C₆ ; cyanoalkyle en C₁-C₆ ; amidoalkyle en C₁-C₆ ; trifluoroalkyle en C₁-C₆ ; sulfonamidoalkyle en C₁-C₆ ; alkyl(C₁-C₆)carboxyalkyle en C₁-C₆ ; alkyl(C₁-C₆)sulfoxyalkyle en C₁-C₆ ; alkyl(C₁-C₆)sulfonalkyle en C₁-C₆ ; alkyl(C₁-C₆)cétoalkyle en C₁-C₆ ; N-alkyl(C₁-C₆)amidoalkyle en C₁-C₆ ou N-alkyl(C₁-C₆)sulfonamidoalkyle en C₁-C₆ ;
- R désigne un atome d'halogène tel que le brome, le chlore ou le fluor, un radical alkyle en C₁-C₆ ou un groupement OZ ou SZ dans lesquels Z désigne un radical alkyle en C₁-C₆, monohydroxyalkyle en C₁-C₆ ou polyhydroxyalkyle en C₂-C₆ ;
étant entendu
- qu'au moins un des groupes -NR₁R₂ et -NR₃R₄ désigne un groupement -NH₂ ;
- que lorsque R₁ et R₂ représentent simultanément un atome d'hydrogène, et que n est égal à 1, alors R est différent d'un radical méthyle ou d'un radical éthyle.

8. 2-hydroxyalkyl paraphénylènediamines selon la revendication 7, caractérisées par le fait qu'elles sont choisies parmi :
- la 2-(β-hydroxyéthyl) 5-chloro paraphénylènediamine,
- la 2-(β-hydroxyéthyl) 5-méthoxy paraphénylènediamine,
- la 2-(β-hydroxyéthyl) 5-méthylthio paraphénylènediamine,
- la 2-(β-hydroxyéthyl) 5-méthyl paraphénylènediamine,
et leurs sels d'addition avec un acide.

9. 2-hydroxyalkyl paraphénylènediamines selon l'une quelconque des revendications 7 ou 8, caractérisées par le fait que les sels d'addition avec un acide sont choisis parmi les chlorhydrates, les bromhydrates, les sulfates, les citrates, les succinates, les tartrates, les lactates et les acétates.

10. Utilisation des 2-hydroxyalkyl paraphénylènediamines de formule (I') telles que définies à l'une quelconque des revendications 7 à 9, à titre de base d'oxydation dans des compositions pour la teinture d'oxydation des fibres kératiniques et en particulier des fibres kératiniques humaines telles que les cheveux.

11. Composition tinctoriale pour la teinture d'oxydation des fibres kératiniques et en particulier des fibres kératiniques humaines telles que les cheveux, caractérisée par le fait qu'elle contient, dans un milieu approprié pour la teinture, au moins une 2-hydroxyalkyl paraphénylènediamine de formule (I') telles que définies à l'une quelconque des revendications 7 à 9, à titre de base d'oxydation.

12. Composition selon la revendication 11, caractérisée par le fait que la ou les 2-hydroxyalkyl paraphénylènediamine de formule (I') et/ou le ou leurs sels d'addition avec un acide représentent de 0,0005 à 12 % en poids du poids total de la composition tinctoriale.

13. Composition selon la revendication 12, caractérisée par le fait que la ou les 2-hydroxyalkyl paraphénylènediamine de formule (I') et/ou le ou leurs sels d'addition avec un acide représentent de 0,005 à 6 % en poids du poids total de la composition tinctoriale.

14. Composition selon l'une quelconque des revendications 11 à 13, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

15. Composition selon l'une quelconque des revendications 11 à 14, caractérisée par le fait qu'elle présente un pH compris entre 3 et 12.

16. Composition selon l'une quelconque des revendications 11 à 15, caractérisée par le fait qu'elle renferme au moins une base d'oxydation additionnelle.

17. Composition selon l'une quelconque des revendications 11 à 16, caractérisée par le fait qu'elle renferme au moins un coupleur et/ou au moins un colorant direct.

18. Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 11 à 17, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

19. Procédé selon la revendication 18, caractérisé par le fait que l'agent oxydant est choisi parmi le peroxyde d'hydrogène, le peroxyde d'urée, les bromates de métaux alcalins, les persels tels que les perborates et persulfates, les peracides, les enzymes.

20. Dispositif à plusieurs compartiments, ou "kit" de teinture à plusieurs compartiments, dont un premier compartiment renferme une composition tinctoriale telle que définie à l'une quelconque des revendications 11 à 17 et un second compartiment renferme une composition oxydante.

## Patentansprüche

1. Verfahren zur Herstellung von 2-Hydroxyalkyl-p-phenylendiaminen, die in 5-Stellung des Benzolrings substituiert oder nicht substituiert sind, und Additionssalzen dieser Verbindungen mit einer Säure,
dadurch gekennzeichnet,
daß es mindestens die folgenden Schritte umfaßt:
Schritt 1: An der Aminogruppe in 1-Stellung des Benzolrings und der Alkoholgruppe in 2-Stellung des Benzolrings eines 2-Hydroxyalkylanilins, das in 5-Stellung des Benzolrings mit einem Halogenatom oder einer C₁₋₆-Alkylgruppe substituiert ist, wird eine Schutzgruppe eingeführt;
Schritt 2: mit der in Schritt 1 hergestellten Verbindung wird in 4-Stellung des Benzolrings eine Nitrierung durchgeführt;
Schritt 3: die Schutzgruppe der Aminogruppe in 1-Stellung des Benzolrings und der Alkoholgruppe in 2-Stellung des Benzolrings der in Schritt 2 hergestellten Verbindung wird entfernt;
Schritt 4: mit der im Schritt 3 hergestellten Verbindung wird eine Reduktion der Nitrogruppe in 4-Stellung durchgeführt, wobei im Falle einer in 5-Stellung halogenierten Verbindung gegebenenfalls das Halogen gleichzeitig entfernt wird.

2. Verfahren nach Anspruch 1, dadurch gekennzeichnet, daß die 2-Hydroxyalkyl-p-phenylendiamine, die in 5-Stellung des Benzolrings substituiert oder nicht substituiert sind, sowie die Additionssalze dieser Verbindungen mit einer Säure der folgenden Formel (I) entsprechen: 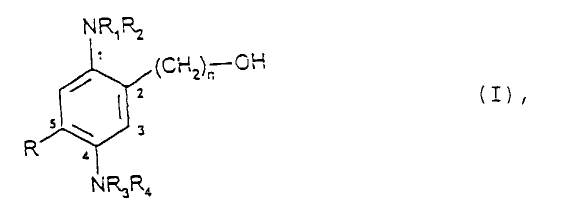 worin bedeuten:
- n eine ganze Zahl im Bereich von 1 bis 4;
- R ein Wasserstoffatom, ein Halogenatom, wie Brom, Chlor oder Fluor, eine C₁₋₆-Alkylgruppe oder eine Gruppe OZ oder SZ, worin Z eine C₁₋₆-Alkylgruppe, eine C₁₋₆-Monohydroxyalkylgruppe oder eine C₂₋₆-Polyhydroxyalkylgruppe bedeutet;
- die Gruppen R₁, R₂, R₃ und R₄, die identisch oder voneinander verschieden sind, Wasserstoff, C₁₋₆-Alkyl, C₁₋₆-Monohydroxyalkyl, C₂₋₆-Polyhydroxyalkyl, C₁₋₆-Alkoxy-C₁₋₆-alkyl, C₁₋₆-Aminoalkyl, C₁₋₆-Mono- oder C₁₋₆-Dihydroxyalkyl-C₁₋₆-aminoalkyl, C₁₋₆-Mono- oder C₁₋₆-Dialkyl-C₁₋₆-aminoalkyl, Aminoalkyl, worin die Aminogruppe mit einer C₁₋₆-Alkylcarbonyl-, einer Carbamoyl- oder einer C₁₋₆-Alkylsulfonylgruppe geschützt ist, C₁₋₆-Carboxyalkyl, C₁₋₆-Cyanoalkyl, C₁₋₆-Amidoalkyl, C₁₋₆-Trifluoralkyl, C₁₋₆-Sulfonamidoalkyl, C₁₋₆-Alkyl-C₁₋₆-carboxyalkyl, C₁₋₆-Alkyl-C₁₋₆-sulfoxyalkyl, C₁₋₆-Alkyl-C₁₋₆-sulfonalkyl, C₁₋₆-Alkyl-C₁₋₆-ketoalkyl, N-C₁₋₆-Alkyl-C₁₋₆-amidoalkyl oder N-C₁₋₆-Alkyl-C₁₋₆-sulfonamidoalkyl;
mit der Maßgabe, daß mindestens eine der Gruppen -NR₁R₂ und -NR₃R₄ die NH₂-Gruppe bedeutet.

3. Verfahren zur Herstellung nach Anspruch 1 oder 2 von Verbindungen, die der folgenden Formel (Ia) entsprechen und Additionssalzen dieser Verbindungen mit einer Säure: 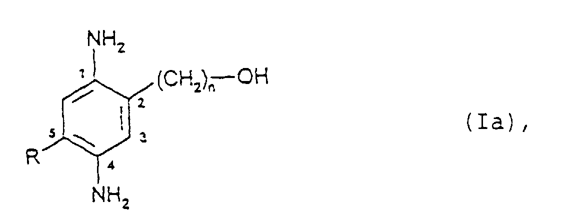 worin bedeuten:
- die Gruppen R₁, R₂, R₃ und R₄ gleichzeitig ein Wasserstoffatom;
- R ein Wasserstoffatom, ein Halogenatom, wie Brom, Chlor oder Fluor, eine C₁₋₆-Alkylgruppe oder eine Gruppe OZ oder SZ, worin Z eine C₁₋₆-Alkylgruppe, eine C₁₋₆-Monohydroxyalkylgruppe oder eine C₂₋₆-Polyhydroxyalkylgruppe bedeutet;
- n eine ganze Zahl im Bereich von 1 bis 4;
wobei das Verfahren die folgenden Schritte umfaßt:
(a) mit einer Verbindung der folgenden Formel (1) : 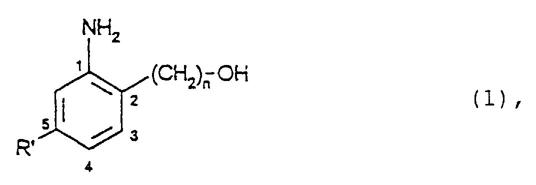 worin R' ein Halogenatom oder eine C₁₋₆-Alkylgruppe bedeutet und n eine ganze Zahl im Bereich von 1 bis 4 ist, wird eine Acetylierung durchgeführt, um eine Verbindung der folgenden Formel (2) herzustellen: 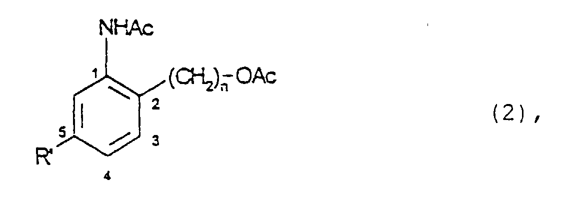 wobei Ac die Acetylgruppe bedeutet und R' und n die oben für die Formel (1) angegebenen Bedeutungen aufweisen;
(b) mit der Verbindung der Formel (2) wird eine Nitrierung in 4-Stellung des Benzolrings durchgeführt, um eine Verbindung der folgenden Formel (3) herzustellen: 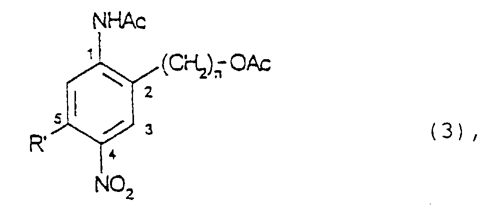 worin R', n und Ac die für die Formel (2) angegebenen Bedeutungen aufweisen;
(c) mit der Verbindung der Formel (3) wird eine Deacetylierung durchgeführt, um die Verbindung der folgenden Formel (4) herzustellen: 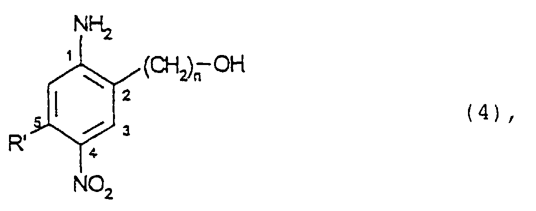 worin R' und n die für die Formel (3) angegebenen Bedeutungen aufweisen;
(d) zur Herstellung der Verbindungen der Formel (Ia), worin R eine Gruppe OZ oder SZ bedeutet, wird die Verbindung der Formel (4), worin R' ein Halogenatom bedeutet, mit einem Alkohol der Formel HOZ oder einem Thiol der Formel HSZ, worin Z die für die Formel (Ia) angegebenen Bedeutungen aufweist, umgesetzt, um eine Verbindung der folgenden Formel (4') herzustellen: 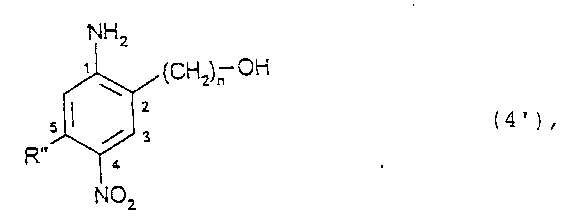 worin R" eine Gruppe OZ oder SZ bedeutet und Z und n die oben für die Formel (Ia) angegebenen Bedeutungen aufweisen; und
(e) mit der Verbindung der Formel (4) oder der Formel (4') wird eine Reduktion durchgeführt, wobei das Reduktionsverfahren unter den folgenden Verfahren ausgewählt wird, wenn die Verbindung der Formel (4) umgesetzt wird, worin R' ein Halogenatom bedeutet:
- zur Herstellung von Verbindungen der Formel (Ia), worin R ein Wasserstoffatom bedeutet, wird eine Reduktion unter Entfernung des Halogenatoms durchgeführt;
- zur Herstellung von Verbindungen der Formel (Ia), worin R ein Halogenatom bedeutet, wird eine Reduktion ohne Entfernen des Halogens durchgeführt.

4. Verfahren zur Herstellung nach Anspruch 1 oder 2 von Verbindungen, die der folgenden Formel (Ib) entsprechen, und den Additionssalzen dieser Verbindungen mit einer Säure: 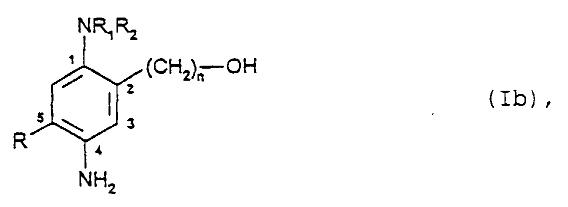 worin bedeuten:
- R ein Wasserstoffatom, ein Halogenatom, wie Brom, Chlor oder Fluor, eine C₁₋₆-Alkylgruppe oder eine Gruppe OZ oder SZ, worin Z eine C₁₋₆-Alkylgruppe, eine C₁₋₆-Monohydroxyalkylgruppe oder eine C₂₋₆-Polyhydroxyalkylgruppe bedeutet;
- die Gruppen R₁ und R₂, die identisch oder voneinander verschieden sind, Wasserstoff, C₁₋₆-Alkyl, C₁₋₆-Monohydroxyalkyl, C₂₋₆-Polyhydroxyalkyl, C₁₋₆-Alkoxy-C₁₋₆-alkyl, C₁₋₆-Aminoalkyl, C₁₋₆-Mono- oder C₁₋₆-Dihydroxyalkyl-C₁₋₆-aminoaikyl, C₁₋₆-Mono- oder C₁₋₆-Dialkyl-C₁₋₆-aminoalkyl, Aminoalkyl, worin die Aminogruppe mit einer C₁₋₆-Alkylcarbonylgruppe, einer Carbamoylgruppe oder einer C₁₋₆-Alkylsulfonylgruppe geschützt ist, C₁₋₆-Carboxyalkyl, C₁₋₆-Cyanoalkyl, C₁₋₆-Amidoalkyl, C₁₋₆-Trifluoralkyl, C₁₋₆-Sulfonamidoalkyl, C₁₋₆-Alkyl-C₁₋₆-carboxyalkyl, C₁₋₆-Alkyl-C₁₋₆-sulfoxyalkyl, C₁₋₆-Alkyl-C₁₋₆-sulfonalkyl, C₁₋₆-Alkyl-C_{1₋6}-ketoalkyl, N-C₁₋₆-Alkyl-C₁₋₆-amidoalkyl oder N-C₁₋₆-Alkyl-C₁₋₆-sulfonamidoalkyl;
- n eine ganze Zahl im Bereich von 1 bis 4,
mit der Maßgabe, daß mindestens eine der Gruppen R₁ und R₂ von Wasserstoff verschieden ist, wobei das Verfahren die folgenden Schritte umfaßt:
(a) mit einer Verbindung der folgenden Formel (1) : 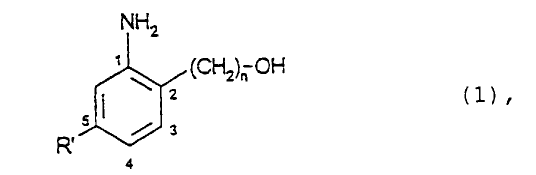 worin R' ein Halogenatom oder eine C₁₋₆-Alkylgruppe bedeutet und n eine ganze Zahl im Bereich von 1 bis 4 ist, wird eine Acetylierung durchgeführt, um eine Verbindung der folgenden Formel (2) herzustellen: 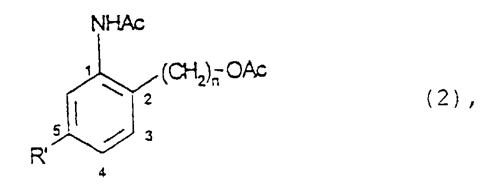 worin Ac die Acetylgruppe bedeutet und R' und n die oben für die Formel (1) angegebenen Bedeutungen aufweisen;
(b) mit der Verbindung der Formel (2) wird eine Nitrierung in 4-Stellung des Benzolrings durchgeführt, um eine Verbindung der folgenden Formel (3) herzustellen: 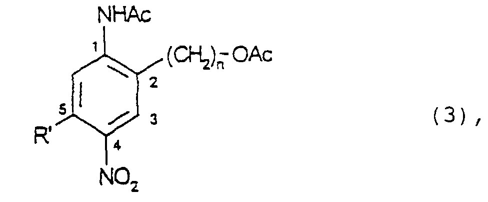 worin R', n und Ac die oben für die Formel (2) angegebenen Bedeutungen aufweisen;
(c) mit der Verbindung der Formel (3) wird eine Deacetylierung durchgeführt, um die Verbindung der folgenden Formel (4) herzustellen: 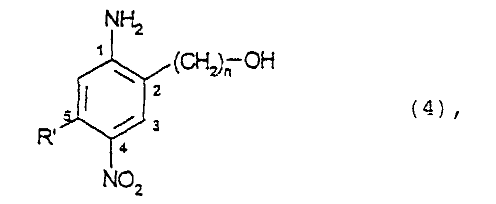 worin R' und n die oben für die Formel (3) angegebenen Bedeutungen aufweisen;
(d) die Aminogruppe der Verbindung der Formel (4) in 1-Stellung des Benzolrings wird mit den Gruppen R₁ und/oder R₂ substituiert, um eine Verbindung der folgenden Formel (5) herzustellen: 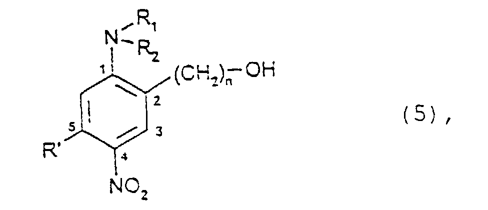 worin R₁, R₂ und n die oben für die Formel (Ib) angegebenen Bedeutungen aufweisen und R' die für die Formel (1) angegebenen Bedeutungen aufweist;
(e) zur Herstellung der Verbindungen der Formel (Ib), worin R eine Gruppe OZ oder SZ bedeutet, wird die Verbindung der Formel (5), worin R' ein Halogenatom bedeutet, mit einem Alkohol der Formel HOZ oder einem Thiol der Formel HSZ, worin Z die oben für die Formel (Ib) angegebenen Bedeutungen aufweist, umgesetzt, um eine Verbindung der folgenden Formel (5') herzustellen: 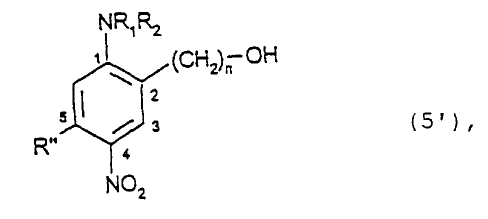 worin R" eine Gruppe OZ oder SZ bedeutet, wobei Z und n die für die Formel (Ib) angegebenen Bedeutungen aufweisen; und
(f) mit der Verbindung der Formel (5) oder der Formel (5') wird eine Reduktion durchgeführt, wobei das Reduktionsverfahren unter den folgenden Verfahren ausgewählt wird, wenn eine Verbindung der Formel (5) umgesetzt wird, worin R' ein Halogenatom bedeutet:
- zur Herstellung der Verbindungen der Formel (Ib), worin R ein Wasserstoffatom bedeutet, wird eine Reduktion unter Entfernung des Halogens durchgeführt;
- zur Herstellung von Verbindungen der Formel (Ib), worin R ein Halogenatom bedeutet, wird eine Reduktion ohne Entfernen des Halogens durchgeführt.

5. Verfahren zur Herstellung nach Anspruch 1 oder 2 von Verbindungen, die der folgenden Formel (Ic) entsprechen, und den Salzen dieser Verbindungen mit einer Säure: 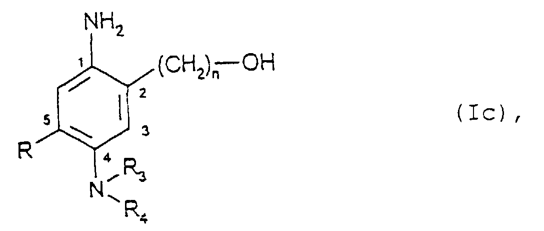 worin bedeuten:
- R ein Wasserstoffatom, ein Halogenatom, wie Brom, Chlor oder Fluor, eine C₁₋₆-Alkylgruppe oder eine Gruppe OZ oder SZ, worin Z eine C₁₋₆-Alkylgruppe, eine C₁₋₆-Monohydroxyalkylgruppe oder eine C₂₋₆-Polyhydroxyalkylgruppe bedeutet;
- die Gruppen R₃ und R₄, die identisch oder voneinander verschieden sind, Wasserstoff, C₁₋₆-Alkyl, C₁₋₆-Monohydroxyalkyl, C₂₋₆-Polyhydroxyalkyl, C₁₋₆-Alkoxy-C₁₋₆-alkyl, C₁₋₆-Aminoalkyl, C₁₋₆-Mono- oder C₁₋₆-Dihydroxyalkyl-C₁₋₆-aminoalkyl, C₁₋₆-Mono- oder C₁₋₆-Dialkyl-C₁₋₆-aminoalkyl, Aminoalkyl, worin die Aminogruppe mit einer C₁₋₆-Alkylcarbonylgruppe, einer Carbamoylgruppe oder einer C₁₋₆-Alkylsulfonylgruppe geschützt ist, C₁₋₆-Carboxyalkyl, C₁₋₆-Cyanoalkyl, C₁₋₆-Amidoalkyl, C₁₋₆-Trifluoralkyl, C₁₋₆-Sulfonamidoalkyl, C₁₋₆-Alkyl-C₁₋₆-carboxyalkyl, C₁₋₆-Alkyl-C₁₋₆-sulfoxyalkyl, C₁₋₆-Alkyl-C₁₋₆-sulfonalkyl, C₁₋₆-Alkyl-C₁₋₆-ketoalkyl, N-C₁₋₆-Alkyl-C₁₋₆-amidoalkyl oder N-C₁₋₆-Alkyl-C₁₋₆-sulfonamidoalkyl;
- n eine ganze Zahl im Bereich von 1 bis 4;
mit der Maßgabe, daß mindestens eine der Gruppen R₃ und R₄ von Wasserstoff verschieden ist, wobei das Verfahren die folgenden Schritte umfaßt:
(a) mit einer Verbindung der folgenden Formel (1) : 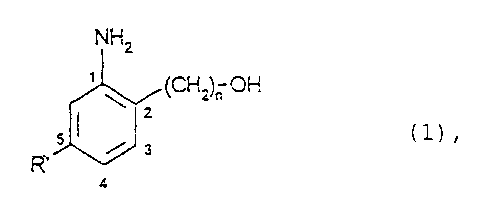 worin R' ein Halogenatom oder eine C₁₋₆-Alkylgruppe bedeutet und n eine ganze Zahl im Bereich von 1 bis 4 ist, wird eine Acetylierung durchgeführt, um eine Verbindung der folgenden Formel (2) herzustellen: 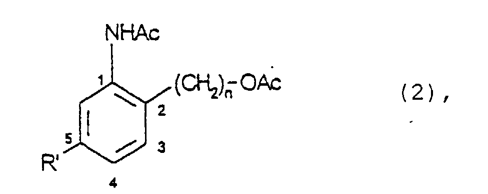 worin Ac die Acetylgruppe bedeutet und R' und n die für die Formel (1) angegebenen Bedeutungen aufweisen;
(b) mit der Verbindung der Formel (2) wird eine Nitrierung in 4-Stellung des Benzolrings durchgeführt, um eine Verbindung der folgenden Formel (3) herzustellen: 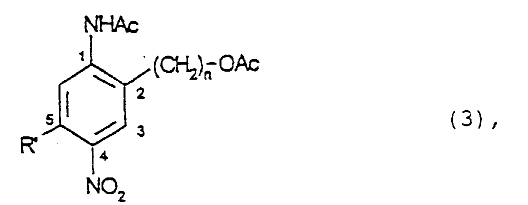 worin R', n und Ac die für die Formel (2) angegebenen Bedeutungen aufweisen;
(c) zur Herstellung von Verbindungen der Formel (Ic), worin R eine Gruppe OZ oder SZ bedeutet, wobei Z die für die Verbindungen der Formel (Ic) angegebenen Bedeutungen aufweist, wird die Verbindung der Formel (3), worin R' ein Halogenatom bedeutet, mit einem Alkohol der Formel HOZ oder einem Thiol der Formel HSZ, wobei Z die oben für die Verbindungen der Formel (Ic) angegebenen Bedeutungen aufweist, umgesetzt, um eine Verbindung der folgenden Formel (3') herzustellen: 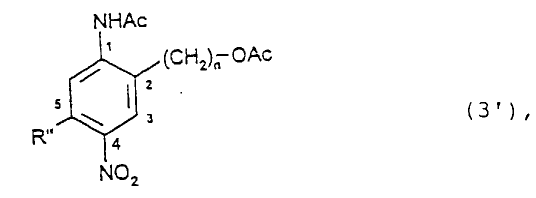 worin Ac die Acetylgruppe und R" eine Gruppe OZ oder SZ bedeutet, wobei Z und n die für die Verbindungen der Formel (Ic) angegebenen Bedeutungen aufweisen;
(d) mit der Verbindung der Formel (3) oder der Formel (3') wird eine Reduktion durchgeführt, wobei das Reduktionsverfahren unter den folgenden Verfahren ausgewählt wird, wenn die Verbindung der Formel (3) umgesetzt wird, worin R' ein Halogenatom bedeutet:
- zur Herstellung von Verbindungen der Formel (Ic), worin R ein Wasserstoffatom bedeutet, wird eine Reduktion unter Entfernen des Halogens durchgeführt;
- zur Herstellung von Verbindungen der Formel (Ic), worin R ein Halogenatom bedeutet, wird eine Reduktion ohne Entfernen des Halogens durchgeführt;
um eine Verbindung der folgenden Formel (6) herzustellen: 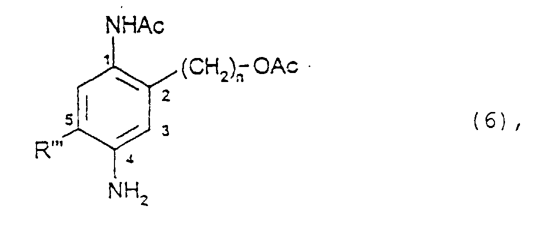 worin die Gruppe R"' R' oder R" bedeutet, wobei R' und R" die für die Verbindungen der Formel (3) und (3') angegebenen Bedeutungen aufweisen, Ac die Acetylgruppe bedeutet und n die für die Verbindungen der Formel (Ic) angegebenen Bedeutungen aufweist;
(e) die Aminogruppe der Verbindung der Formel (6) in 4-Stellung des Benzolrings wird mit den Gruppen R₃ und/oder R₄ substituiert, um eine Verbindung der folgenden Formel (7) herzustellen: 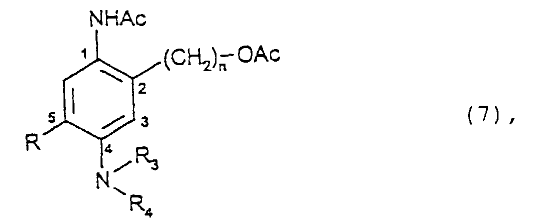 worin R, R₃, R₄, Ac und n die für die Formel (Ic) und (2) angegebenen Bedeutungen aufweisen; und
(f) mit der Verbindung der Formel (7) wird eine Deacetylierung durchgeführt, um die Verbindung der Formel (Ic) herzustellen.

6. Verfahren nach einem der vorhergehenden Ansprüche, dadurch gekennzeichnet, daß die Additionssalze mit einer Säure unter den Hydrochloriden, Hydrobromiden, Sulfaten, Citraten, Succinaten, Tartraten, Lactaten und Acetaten ausgewählt sind.

7. 2-Hydroxyalkyl-p-phenylendiamine der folgenden Formel (I') und die Additionssalze dieser Verbindungen mit einer Säure: 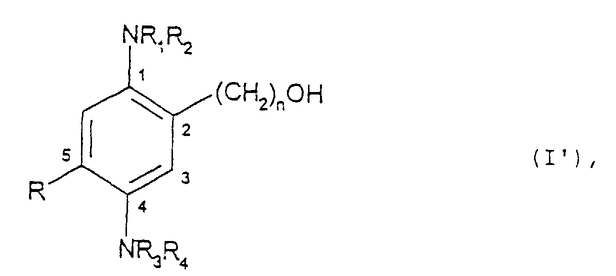 worin bedeuten:
- n eine ganze Zahl im Bereich von 1 bis 4;
- die Gruppen R₁, R₂, R₃ und R₄, die identisch oder voneinander verschieden sind, Wasserstoff, C₁₋₆-Alkyl, C₁₋₆-Monohydroxyalkyl, C₂₋₆-Polyhydroxyalkyl, C₁₋₆-Alkoxy-C₁₋₆-alkyl, C₁₋₆-Aminoalkyl, C₁₋₆-Mono- oder C₁₋₆-Dihydroxyalkyl-C₁₋₆-aminoalkyl, C₁₋₆-Mono- oder C₁₋₆-Dialkyl-C₁₋₆-aminoalkyl, Aminoalkyl, worin das Amin mit einer C₁₋₆-Alkylcarbonylgruppe, einer Carbamoylgruppe oder einer C₁₋₆-Alkylsulfonylgruppe geschützt ist, C₁₋₆-Carboxyalkyl, C₁₋₆-Cyanoalkyl, C₁₋₆-Amidoalkyl, C₁₋₆-Trifluoralkyl, C₁₋₆-Sulfonamidoalkyl, C₁₋₆-Alkyl-C₁₋₆-carboxyalkyl, C₁₋₆-Alkyl-C₁₋₆-sulfoxyalkyl, C₁₋₆-Alkyl-C₁₋₆-sulfonalkyl, C₁₋₆-Alkyl-C₁₋₆-ketoalkyl, N-C₁₋₆-Alkyl-C₁₋₆-amidoalkyl oder N-C₁₋₆-Alkyl-C₁₋₆-sulfonamidoalkyl;
- R ein Halogenatom, wie Brom, Chlor oder Fluor, eine C₁₋₆-Alkylgruppe oder eine Gruppe OZ oder SZ, worin Z eine C₁₋₆-Alkylgruppe, eine C₁₋₆-Monohydroxyalkylgruppe oder eine C₂₋₆-Polyhydroxyalkylgruppe bedeutet;
mit der Maßgabe, daß
- mindestens eine der Gruppen -NR₁R₂ und -NR₃R₄ die NH₂-Gruppe bedeutet; und
- R von Methyl oder Ethyl verschieden ist, wenn R₁ und R₂ gleichzeitig Wasserstoff bedeuten und n 1 ist.

8. 2-Hydroxyalkyl-p-phenylendiamine nach Anspruch 7, dadurch gekennzeichnet, daß sie ausgewählt sind unter:
- 2-(β-Hydroxyethyl)-5-chlor-p-phenylendiamin,
- 2-(β-Hydroxyethyl)-5-methoxy-p-phenylendiamin,
- 2-(β-Hydroxyethyl)-5-methylthio-p-phenylendiamin,
- 2-(β-Hydroxyethyl)-5-methyl-p-phenylendiamin, und den Additionssalzen dieser Verbindungen mit einer Säure.

9. 2-Hydroxyalkyl-p-phenylendiamine nach einem der Ansprüche 7 oder 8, dadurch gekennzeichnet, daß die Additionssalze mit einer Säure unter den Hydrochloriden, Hydrobromiden, Sulfaten, Citraten, Succinaten, Tartraten, Lactaten und Acetaten ausgewählt sind.

10. Verwendung von 2-Hydroxyalkyl-p-phenylendiaminen der Formel (I') nach einem der Ansprüche 7 bis 9 als Oxidationsbasen in Zusammensetzungen zum oxidativen Färben von Keratinfasern und insbesondere menschlichen Keratinfasern, wie dem Haar.

11. Färbemittelzusammensetzung zum oxidativen Färben von Keratinfasern und insbesondere menschlichen Keratinfasern, wie dem Haar, dadurch gekennzeichnet, das sie in einem zum Färben geeigneten Medium mindestens ein 2-Hydroxyalkyl-p-phenylendiamin der Formel (I') nach einem der Ansprüche 7 bis 9 als Oxidationsbase enthält.

12. Zusammensetzung nach Anspruch 11, dadurch gekennzeichnet, daß das 2-Hydroxyalkyl-p-phenylendiamin oder die 2-Hydroxyalkyl-p-phenylendiamine der Formel (I') und/oder das oder die Additionssalze dieser Verbindungen mit einer Säure 0,0005 bis 12 Gew.-% des Gesamtgewichts der Färbemittelzusammensetzung ausmachen.

13. Zusammensetzung nach Anspruch 12, dadurch gekennzeichnet, daß das 2-Hydroxyalkyl-p-phenylendiamin oder die 2-Hydroxyalkyl-p-phenylendiamine der Formel (I') und/oder das oder die Additionssalze dieser Verbindungen mit einer Säure 0,005 bis 6 Gew.-% des Gesamtgewichts der Färbemittelzusammensetzung ausmachen.

14. Zusammensetzung nach einem der Ansprüche 11 bis 13, dadurch gekennzeichnet, daß das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

15. Zusammensetzung nach einem der Ansprüche 11 bis 14, dadurch gekennzeichnet, daß sie einen pH-Wert im Bereich von 3 bis 12 aufweist.

16. Zusammensetzung nach einem der Ansprüche 11 bis 15, dadurch gekennzeichnet, daß sie mindestens eine zusätzliche Oxidationsbase enthält.

17. Zusammensetzung nach einem der Ansprüche 11 bis 16, dadurch gekennzeichnet, daß sie mindestens einen Kuppler und/oder mindestens einen Direktfarbstoff enthält.

18. Verfahren zum Färben von Keratinfasern und insbesondere menschlichen Keratinfasern wie dem Haar, dadurch gekennzeichnet, daß auf die Fasern mindestens eine Färbemittelzusammensetzung nach einem der Ansprüche 11 bis 17 aufgetragen wird und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

19. Verfahren nach Anspruch 18, dadurch gekennzeichnet, daß das Oxidationsmittel unter Wasserstoffperoxid, Harnstoffperoxid, Alkalimetallbromaten, Salzen von Persäuren, wie Perboraten und Persulfaten, Persäuren und Enzymen ausgewählt ist.

20. Vorrichtung mit mehreren Abteilungen oder Kit zum Färben mit mehreren Abteilungen, wobei eine erste Abteilung eine Färbemittelzusammensetzung nach einem der Ansprüche 11 bis 17 und eine zweite Abteilung eine oxidierende Zusammensetzung enthält.

## Claims

1. Process for the synthesis of 2-hydroxyalkyl-para-phenylenediamines which are substituted or unsubstituted in position 5 of the benzene ring, and of addition salts thereof with an acid, characterized in that it comprises at least the following steps:
Step No. 1: the amine function in position 1 of the benzene ring and the alcohol function in position 2 of the benzene ring of a 2-hydroxyalkylaniline substituted in position 5 of the benzene ring with a halogen atom or with a C₁-C₆ alkyl radical, are protected;
Step No. 2: a reaction of nitration in position 4 of the benzene ring is carried out on the compound obtained in step No. 1;
Step No. 3: deprotection of the amine function in position 1 of the benzene ring and of the alcohol function in position 2 of the benzene ring of the compound obtained in step No. 2 is carried out;
Step No. 4: a reaction of reduction of the nitro group in position 4, which may or may not be accompanied, in the case of a 5-halo compound, by a dehalogenation, is carried out on the compound obtained in step No. 3.

2. Process according to Claim 1, characterized in that the 2-hydroxyalkyl-para-phenylenediamines which are substituted or unsubstituted in position 5 on the benzene ring, and the addition salts thereof with an acid, correspond to formula (I) below: 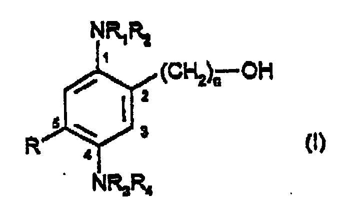 in which:
- n is an integer between 1 and 4 inclusive;
- R denotes a hydrogen atom or halogen atom such as bromine, chlorine or fluorine, a C₁-C₆ alkyl radical or a group OZ or SZ in which Z denotes a C₁-C₆ alkyl, C₁-C₆ monohydroxyalkyl or C₂-C₆ polyhydroxyalkyl radical;
- R₁, R₂, R₃ and R₄, which may be identical or different, denote a hydrogen atom, a C₁-C₆ alkyl, C₁-C₆ monohydroxyalkyl, C₂-C₆ polyhydroxyalkyl, (C₁-C₆)alkoxy(C₁-C₆)alkyl, C₁-C₆ aminoalkyl, mono- or dihydroxy(C₁-C₆)alkylamino(C₁-C₆)alkyl; mono- or di(C₁-C₆)alkylamino(C₁-C₆)alkyl radicals; aminoalkyl radical in which the amine is protected with a (C₁-C₆)alkylcarbonyl, carbamyl or (C₁-C₆)alkylsulphonyl radical; C₁-C₆ carboxyalkyl; cyano(C₁-C₆)alkyl; C₁-C₆ amidoalkyl; C₁-C₆ trifluoroalkyl; C₁-C₆ sulphonamidoalkyl; (C₁-C₆)alkylcarboxy (C₁-C₆)alkyl; (C₁-C₆)alkylsulphoxy(C₁-C₆)alkyl; (C₁-C₆)alkylsulphon (C₁-C₆)alkyl; (C₁-C₆)alkylketo(C₁-C₆)alkyl; N(C₁-C₆)alkylamido(C₁-C₆)alkyl or N-(C₁-C₆)alkylsulphonamido(C₁-C₆)alkyl radical;
it being understood that at least one of the groups -NR₁R₂ and -NR₃R₄ denotes an -NH₂ group.

3. Process for the synthesis, according to Claim 1 or 2, of compounds corresponding to formula (Ia) below, and of the addition salts thereof with an acid: 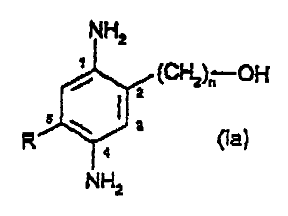 in which:
- R₁, R₂, R₃ and R₄ simultaneously denote a hydrogen atom;
- R denotes a hydrogen atom or halogen atom such as bromine, chlorine or fluorine, a C₁-C₆ alkyl radical or a group Oz or SZ in which Z denotes a C₁-C₆ alkyl, C₁-C₆ monohydroxyalkyl or C₂-C₆ polyhydroxyalkyl radical;
- n is an integer between 1 and 4 inclusive;
the said process comprising the following steps:
(a) an acetylation reaction is carried out on a compound of formula (1) below: 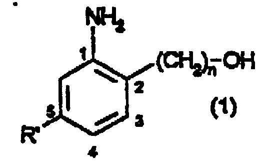 in which R' denotes a halogen atom or a C₁-C₆ alkyl radical and n is an integer between 1 and 4 inclusive, to give a compound of formula (2) below: 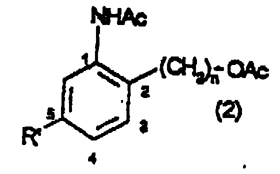 in which Ac denotes an acetyl group and R' and n have the same meanings as those given above for formula (1);
(b) a nitration reaction in position 4 of the benzene ring is carried out on the compound of formula (2), to give a compound of formula (3) below: 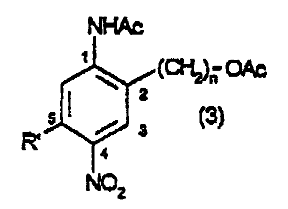 in which R', n and Ac have the same meanings as those given above for formula (2);
(c) a deacetylation reaction is carried out on the compound of formula (3), to give the compound of formula (4) below: 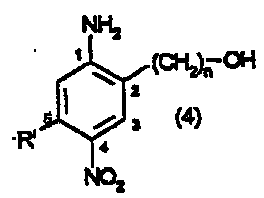 in which R' and n have the same meanings as those given above for formula (3); and then
(d) to obtain the compounds of formula (Ia) in which R denotes a group OZ or SZ, the compound of formula (4), in which R' denotes a halogen atom, is reacted with an alcohol of formula HOZ or a thiol of formula HSZ in which Z has the same meaning as that given for formula (Ia), to give a compound of formula (4') below: 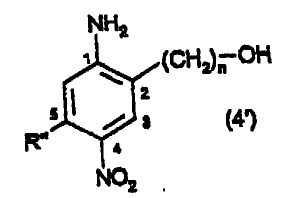 in which R" denotes a group OZ or SZ, Z and n having the same meanings as those given above for formula (Ia);
(e) a reduction reaction is carried out on the compound of formula (4) or of formula (4'),
it being understood that when the compound of formula (4) in which R' denotes a halogen atom is reacted, the reduction method is chosen from the following processes:
- to obtain the compounds of formula (Ia) in which R denotes a hydrogen atom, a dehalogenating reduction reaction is carried out;
- to obtain the compounds of formula (Ia) in which R denotes a halogen atom, a non-dehalogenating reduction reaction is carried out.

4. Process for the synthesis, according to Claim 1 or 2, of compounds corresponding to formula (Ib) below, and of the addition salts thereof with an acid; 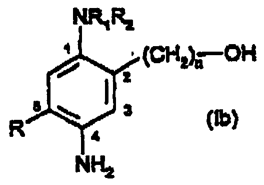 in which:
- R denotes a hydrogen atom or halogen atom such as bromine, chlorine or fluorine, a C₁-C₆ alkyl radical or a group OZ or SZ in which Z denotes a C₁-C₆ alkyl, C₁-C₆ monohydroxyalkyl or C₂-C₆ polyhydroxyalkyl radical;
- R₁ and R₂, which may be identical or different, denote a hydrogen atom, a C₁-C₆ alkyl, C₁-C₆ monohydroxyalkyl, C₂-C₆ polyhydroxyalkyl, (C₁-C₆)alkoxy(C₁-C₆)alkyl, C₁-C₆ aminoalkyl, mono- or dibydroxy(C₁-C₆)alkylamino(C₁-C₆)alkyl; mono- or di(C₁-C₆)alkylamino(C₁-C₆)alkyl radicals; aminoalkyl radical in which the amine is protected with a (C₁-C₆)alkylcarbonyl, carbamyl or (C₁-C₆)alkylsulphonyl radical; C₁-C₆ carboxyalkyl; cyano(C₁-C₆)alkyl; C₁-C₆ amidoalkyl; C₁-C₆ trifluoroalkyl; C₁-C₆ sulphonamidoalkyl; (C₁-C₆)alkylcarboxy(C₁-C₆)alkyl; (C₁-C₆)alkylsulphoxy(C₁-C₆)alkyl; (C₁-C₆)alkylsulphon(C₁-C₆)alkyl; (C₁-C₆)alkylketo(C₁-C₆)alkyl; N-(C₁-C₆)-alkylamido(C₁-C₆)alkyl or N-(C₁-C₆)alkylsulphonamido(C₁-C₆)alkyl radical;
- n is an integer between 1 and 4 inclusive;
it being understood that at least one of the radicals R₁ and R₂ is other than a hydrogen atom; the said process comprising the following steps:
(a) an acetylation reaction is carried out on a compound of formula (1) below: 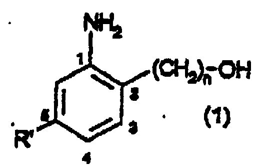 in which R' denotes a halogen atom or a C₁-C₆ alkyl radical and n is an integer between 1 and 4 inclusive, to give a compound of formula (2) below: 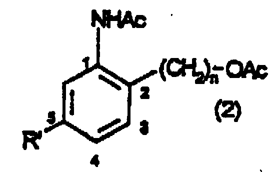 in which Ac denotes an acetyl group and R' and n have the same meanings as those given above for formula (1);
(b) a nitration reaction in position 4 of the benzene ring is carried out on the compound of formula (2), to give a compound of formula (3) below: 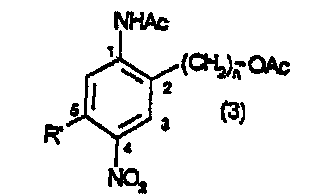 in which R', n and Ac have the same meanings as those given above for formula (2) ;
(c) a deacetylation reaction is carried out on the compound of formula (3), to give the compound of formula (4) below: 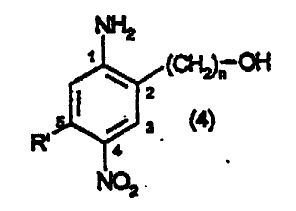 in which R' and n have the same meanings as those given above for formula (3);
(d) substitution of the amine function in position 1 of the benzene ring with the radicals R₁ and/or R₂ is carried out on the compound of formula (4), to give a compound of formula (5) below: 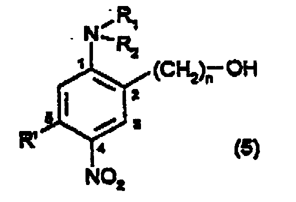 in which R₁, R₂ and n have the same meanings as those given above for formula (Ib) and R' has the same meaning as that given above for formula (1); and then
(e) to obtain the compounds of formula (Ib) in which R denotes a group OZ or SZ, the compound of formula (5), in which R' denotes a halogen atom, is reacted with an alcohol of formula HOZ or a thiol of formula HSZ in which Z has the same meaning as those given above for formula (Ib), to give a compound of formula (5') below: 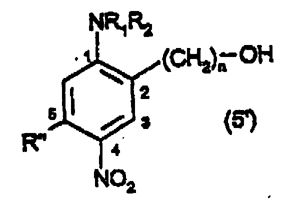 in which R" denotes a group OZ or SZ in which Z and n have the same meanings as those given for formula (Ib) above;
(f) a reduction reaction is carried out on the compound of formula (5) or of formula (5'), it being understood that when a compound of formula (5) in which R' denotes a halogen atom is reacted, the reduction method, is chosen from the following processes:
- to obtain the compounds of formula (Ib) in which R denotes a hydrogen atom, a dehalogenating reduction reaction is carried out;
- to obtain the compounds of formula (Ib) in which R denotes a halogen atom, a non-dehalogenating reduction reaction is carried out.

5. Process for the synthesis, according to Claim 1 or 2, of compounds corresponding to formula (Ic) below, and of the addition salts thereof with an acid: 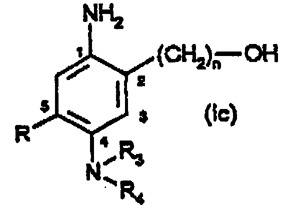 in which:
- R denotes a hydrogen atom or halogen atom such as bromine, chlorine or fluorine, a C₁-C₆ alkyl radical or a group OZ or SZ in which Z denotes a C₁-C₆ alkyl, C₁-C₆ monohydroxyalkyl or C₂-C₆ polyhydroxyalkyl radical;
- R₃ and R₄, which may be identical or different, denote a hydrogen atom, a C₁-C₆ alkyl, C₁-C₆ monohydroxyalkyl, C₂-C₆ polyhydroxyalkyl, (C₁-C₆)alkoxy (C₁-C₆)alkyl, C₁-C₆ aminoalkyl, mono- or dihydroxy(C₁-C₆)alkylamino(C₁-C₆)alkyl; mono- or di(C₁-C₆)alkylamino(C₁-C₆)alkyl radicals; aminoalkyl radical in which the amine is protected with a (C₁-C₆)alkylcarbonyl, carbamyl or (C₁-C₆)alkylsulphonyl radical; C₁-C₆ carboxyalkyl; cyano(C₁-C₆)alkyl: C₁-C₆ amidoalkyl; C₁-C₆ trifluoroalkyl; C₁-C₆ sulphonamidoalkyl; (C₁-C₆)alkylcarboxy(C₁-C₆)alkyl; (C₁-C₆)alkylsulphoxy(C₁-C₆)alkyl; (C₁-C₆)alkylsulphon(C₁-C₆)alkyl; (C₁-C₆)alkylketo(C₁-C₆)alkyl; N-(C₁-C₆)-alkylamido(C₁-C₆)alkyl or N-(C₁-C₆)alkylsulphonamido(C₁-₆)alkyl radical;
- n is an integer between 1 and 4 inclusive;
it being understood that at least one of the radicals - R₃ and R₄ is other than a hydrogen atom;
the said process comprising the following steps:
(a) an acetylation reaction is carried out on a compound of formula (1) below: 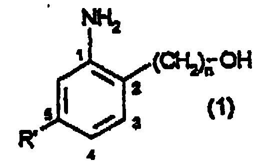 in which R' denotes a halogen atom or a C₁-C₆ alkyl radical and n is an integer between 1 and 4 inclusive, to give a compound of formula (2) below: 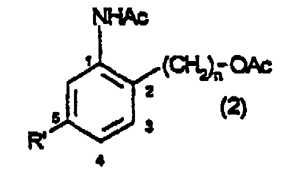 in which Ac denotes an acetyl group and R' and n have the same meanings as those given above for formula (1);
(b) a nitration reaction in position 4 of the benzene ring is carried out on the compound of formula (2), to give a compound of formula (3) below: 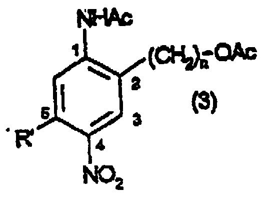 in which R', n and Ac have the same meanings as those given above for formula (2);
(c) to obtain the compounds of formula (Ic) in which R denotes a group OZ or SZ, Z having the same meanings as those given above for the compounds of formula (Ic), the compound of formula (3) in which R' denotes a halogen atom is reacted with an alcohol of formula HOZ or a thiol of formula HSZ in which Z has the same meanings as those given above for the compounds of formula (Ic), to give a compound of formula (3') below: 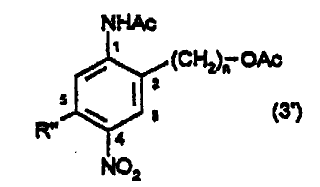 in which Ac represents an acetyl group and R" denotes a group OZ or SZ, Z and n having the same meanings as those given above for the compounds of formula (Ic);
(d) a reduction reaction is carried out on the compound of formula (3) or of formula (3') it being understood that when the compound of formula (3) in which R' denotes a halogen atom is reacted, the reduction method is chosen from the following processes:
- to obtain the compounds of formula (Ic) in which R denotes a hydrogen atom, a dehalogenating reduction reaction is carried out;
- to obtain the compounds of formula (Ic) in which R denotes a halogen atom, a non-dehalogenating reduction reaction is carried outs
to give a compound of formula (6) below: 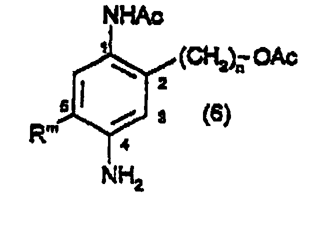 in which R"' denotes R' or R", R' and R" having the same meanings as those given above for the compounds of formulae (3) and (3'), Ac represents an acetyl group and n having the same meanings as those given above for the compounds of formula (Ic);
(e) substitution of the amine function in position 4 of the benzene ring with the radicals R₃ and/or R₄ is carried out on the compound of formula (6), to give a compound of formula (7) below: 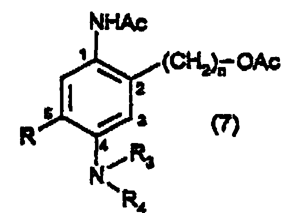 in which R, R₃, R₄, Ac and n have the same meanings as those given above for formulae (Ic) and (2);
(f) a deacetylation reaction is carried out on the compound of formula (7) to give the compound of formula (Ic).

6. Process according to any one of the preceding claims, characterized in that the addition salts with an acid are chosen from the hydrochlorides, hydrobromides, sulphates, citrates, succinates, tartrates, lactates and acetates.

7. 2-Hydroxyalkyl-para-phenylenediamines corresponding to formula (I') below, and the addition salts thereof with an acid: 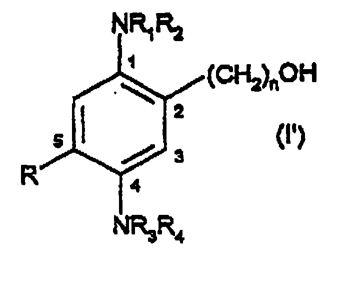 in which:
- n is an integer between 1 and 4 inclusive;
- R₁, R₂, R₃ and R₄, which may be identical or different, denote a hydrogen atom, a C₁-C₆ alkyl, C₁-C₆ monohydroxyalkyl, C₂-C₆ polyhydroxyalkyl, (C₁-C₆)alkoxy(C₁-C₆)alkyl, C₁-C₆ aminoalkyl, mono- or dihydroxy(C₁-C₆)alkylamino(C₁-C₆)alkyl; mono- or di(C₁-C₆)alkylamino(C₁-C₆)alkyl radicals; aminoalkyl radical in which the amine is protected with a (C₁-C₆)alkylcarbonyl, carbamyl or (C₁-C₆)alkylsulphonyl radicals C₁-C₆ carboxyalkyl; cyano(C₁-C₆)alkyl; C₁-C₆ amidoalkyl; C₁-C₆ trifluoroalkyl; C₁-C₆ sulphonamidoalkyl; (C₁-C₆)alkylcarboxy(C₁-C₆)alkyl; (C₁-C₆)alkylsulphoxy(C₁-C₆)alkyl; (C₁-C₆)alkylsulphon(C₁-C₆)alkyl; (C₁-C₆)alkylketo(C₁-C₆)alkyl; N-(C₁-C₆)alkylamido (C₁-C₆)alkyl or N-(C₁-C₆)alkylsulphonamido(C₁-C₆)alkyl radical;
- R denotes a halogen atom such as bromine, chlorine or fluorine, a C₁-C₆ alkyl radical or a group OZ or SZ in which Z denotes a C₁-C₆ alkyl, C₁-C₆ monohydroxyalkyl or C₂-C₆ polyhydroxyalkyl radical;
it being understood
- that at least one of the groups -NR₁R₂ and -NR₃R₄ denotes an -NH₂ group;
- that when R₁ and R₂ simultaneously represent a hydrogen atom, and when n is equal to l, then R is other than a methyl radical or an ethyl radical.

8. 2-Hydroxyalkyl-para-phenylenediamines according to Claim 7, characterized in that they are chosen from:
- 2-(β-hydroxyethyl)-5-chloro-para-phenylenediamine,
- 2-(β-hydroxyethyl)-5-methoxy-para-phenylenediamine,
- 2-(β-hydroxyethyl)-5-methyltho-para-phenylene-diamine,
- 2-(β-hydroxyethyl)-5-methyl-para-phenylenediamine,
and the addition salts thereof with an acid.

9. 2-Hydroxyalkyl-para-phenylenediamines according to either of Claims 7 and 8, characterized in that the addition salts with an acid are chosen from the hydrochlorides, hydrobromides, sulphates, citrates, succinates, tartrates, lactates and acetates.

10. Use of the 2-hydroxyalkyl-para-phenylenediamines of formula (I') as defined in any one of Claims 7 to 9, as oxidation bases in compositions for the oxidation dyeing of keratin fibres, and in particular human keratin fibres such as the hair.

11. Dye composition for the oxidation dyeing of keratin fibres, and in particular human keratin fibres such as the hair, characterized in that it contains, in a medium which is suitable for dyeing, at least one 2-hydroxyalkyl-para-phenylenediamine of formula (I') as defined in any one of Claims 7 to 9, as oxidation base.

12. Composition according to Claim 11, characterized in that the 2-hydroxyalkyl-para-phenylenediamine of formula (I') and/or the addition salt(s) thereof with an acid represent(s) from 0.0005 to 12% by weight relative to the total weight of the dye composition.

13. Composition according to Claim 12, characterized in that the 2-hydroxyalkyl-para-phenylenediamine {9) of formula (I') and/or the addition salt(s) thereof with an acid represent(s) from 0.005 to 6% by weight relative to the total weight of the dye composition.

14. Composition according to any one of Claims 11 to 13, characterized in that the medium which is suitable for dyeing (or support) consists of water or a mixture of water and at least one organic solvent chosen from C₁-C₄ lower alkanols, glycerol, glycols and glycol ethers, aromatic alcohols, similar products and mixtures thereof.

15. Composition according to any one of Claims 11 to 14, characterized in that it has a pH of between 3 and 12.

16. Composition according to any one of Claims 11 to 15, characterized in that it contains at least one additional oxidation base.

17. Composition according to any one of Claims 11 to 16, characterized in that it contains at least one coupler and/or at least one direct dye.

18. Process for dyeing keratin fibres, and in particular human keratin fibres such as the hair, characterized in that at least one dye composition as defined in any one of Claims 11 to 17 is applied to these fibres, and in that the colour is developed at acidic, neutral or alkaline pH using an oxidizing agent which is added to the dye composition only at the time of use, or which is present in an oxidizing composition that is applied simultaneously or sequentially in a separate manner.

19. Process according to Claim 18, characterized in that the oxidizing agent is chosen from hydrogen peroxide, urea peroxide, alkali metal bromates, persalts such as perborates and persulphates, peracids and enzymes.

20. Multi-compartment dyeing device or multi-compartment dyeing "kit", a first compartment of which contains a dye composition as defined in any one of Claims 11 to 17, and a second compartment of which contains an oxidizing composition.
